# FASCICULE DE BREVET EUROPEEN

(11) **EP 0 409 728 B1**
(45) Date de publication et mention de la délivrance du brevet: **01.03.1995**
(21) Numéro de dépôt: 90402072.4
(22) Date de dépôt: 18.07.1990
(51) Int. Cl.: C07C 69/76, C07C 323/62, C07C 317/44, A61K 31/235, A61K 7/48

(54) **Esters bi-aromatiques, leur procédé de préparation et leur utilisation en médecine humaine ou vétérinaire et en cosmétique**
Bi-aromatische Ester, Verfahren zu ihrer Herstellung und ihre Verwendung in der menschlichen oder tierischen Heilkunde und in der Kosmetik
Bi-aromatic esters, process for their preparation and their use in human or animal medicine and in cosmetics

(30) Priorité: 18.07.1989 FR 8909652
(43) Date de publication de la demande: 23.01.1991
(73) Titulaire: CENTRE INTERNATIONAL DE RECHERCHES DERMATOLOGIQUES GALDERMA, ( CIRD GALDERMA), 06560 Valbonne (FR)
(72) Inventeur: Shroot, Braham, Villa 35, F-06600 Antibes (FR); Bernardon, Jean-Michel, F-06650 Nice (FR)
(74) Mandataire: Nony, Michel

(56) Documents cités:
- EP-A- 0 232 199
- EP-A- 0 325 540
- US-A- 4 000 144
- US-A- 4 038 250

## Description

La présente invention a pour objet de nouveaux esters bi-aromatiques, leur procédé de préparation et leur utilisation en médecine humaine et vétérinaire et en cosmétique.

Ces nouveaux esters bi-aromatiques trouvent une application dans le traitement topique et systémique des affections dermatologiques liées à un désordre de la kératinisation (différenciation-prolifération) et d'affections dermatologiques, ou autres, à composantes inflammatoires et/ou immunoallergiques et dans les maladies de dégénérescence du tissu conjonctif, et présentent une activité antitumorale. En outre, ces dérivés peuvent être utilisés dans le traitement de l'atopie, qu'elle soit cutanée ou respiratoire et du psoriasis rhumatoïde.

Ils trouvent également une application dans le domaine ophtalmologique, notamment dans le traitement des cornéopathies. Le demande de brevet européen n° 232.199 décrit certains composés benzamido aromatiques également utiles dans ce domaine.

Les esters bi-aromatiques selon l'invention sont choisis parmi les suivants :
Acide 4-[3-(1-adamantyl)-4-méthoxybenzoyloxy]- 3-fluorobenzoïque,
Acide 4-[3-(1-adamantyl)-4-méthoxybenzoyloxy]- 3-méthoxybenzoïque,
Acide 4-[3-(1-adamantyl)-4-méthoxybenzoyloxy]-isophtalique,
Acide 4-[3-(1-adamantyl)-4-acétoxybenzoyloxy]-benzoïque,
Acide 4-[3-(1-adamantyl)-4-(carboxyméthylèneoxy) benzoyloxy] benzoïque,
Acide 4-[3-(1-adamantyl)-4-(2,3-dihydroxy-propyloxy) benzoyloxy] benzoïque,
Acide 4-[3-(1-adamantyl)-4-(méthoxycarbonylméthyloxy) benzoyloxy] benzoïque, et ceux répondant à la formule générale suivante : 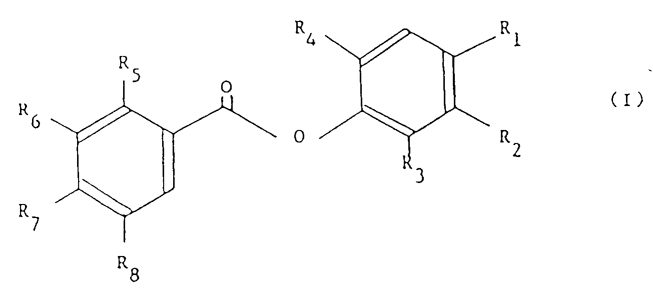 dans laquelle:
R₁ représente le groupe OH, le radical -CH₃,
-CH₂OH, -CH(OH)CH₃, -COOR₉, 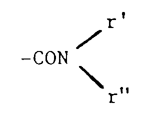 ou SO₂R₁₀,
R₉ représente un atome d'hydrogène, un radical alkyle ayant de 1 à 6 atomes de carbone ou un radical mono ou polyhydroxyalkyle,
R₁₀ représentant le groupe OH, un radical alkyle ayant de 1 à 6 atomes de carbone ou le radical 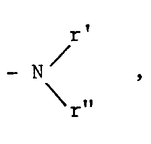 r' et r'' représentant un atome d'hydrogène, un radical alkyle ayant de 1 à 6 atomes de carbone, aryle, aralkyle, mono ou polyhydroxyalkyle, ou r' et r'' pris ensemble forment un hétérocycle,
R₂ représente un atome d'hydrogène, un radical alkyle ayant de 1 à 6 atomes de carbone, le radical OR₉ , un atome de fluor ou le radical -CF₃,
R₃ , R₄ et R₅ représentent un atome d'hydrogène, un atome de fluor, le groupe OH, le radical -CH₃, -OCH₃, -CF₃, -COOH ou -CH₂OH, R₆ et R₈ représentent un atome d'hydrogène, un radical alkyle α-substitué ayant de 3 à 15 atomes de carbone, un radical alkyle α-α'-disubstitué ayant de 4 à 12 atomes de carbone, un radical cycloalkyle ayant de 3 à 12 atomes de carbone, un radical cycloalkyle mono ou polycyclique ayant de 5 à 12 atomes de carbone dont le carbone de liaison est trisubstitué, le radical -SR₁₁, -SOR₁₁ ou -SO₂R₁₁,
R₁₁ représentant un radical alkyle ayant de 1 à 6 atomes de carbone ou un radical cycloalkyle, R₆ et R₈ ne pouvant pas simultanément représenter un atome d'hydrogène,
R₇ représente un atome d'hydrogène, un radical alkyle ayant de 1 à 6 atomes de carbone, un radical alkényle, un radical alkényloxy, le radical OR₁₂, SR₁₃ , SO R₁₄ ou SO₂R₁₄,
R₁₂ représentant un atome d'hydrogène, un radical alkyle ayant de 1 à 6 atomes de carbone, un radical alkényle, un radical mono ou polyhydroxyalkyle ou le radical -(CH₂)ₙ-COR₁₅
n étant 0,1 ou 2 et R₁₅ représentant un atome d'hydrogène, le groupe OH, un radical alkyle ayant de 1 à 6 atomes de carbone ou un radical alkoxy ayant de 1 à 6 atomes de carbone,
R₁₃ représentant un atome d'hydrogène, un radical alkyle ayant de 1 à 6 atomes de carbone ou un radical aralkyle,
R₁₄ représentant le groupe OH, un radical alkyle ayant de 1 à 6 atomes de carbone ou un radical aralkyle,
sous réserve que lorsque R₁ représente -CH₂OH, -CH(OH)CH₃, -COOR₉ ou 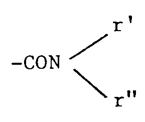 et R₂ représente un atome d'hydrogène alors :
(i) soit R₃ et R₄ sont différents d'un atome d'hydrogène ou du radical -CH₃ ,
(ii) soit R₇ est différent du radical OR₁₂ et R₆ ou R₈ est un radical cycloalkyle ayant plus de 7 atomes de carbone,
(iii) soit R₇ représente le radical OR₁₂ , mais R₆ et R₈ sont alors différents d'un atome d'hydrogène,
(iv) soit R₇ représente le radical OR₁₂ , mais alors R₅ est différent d'un atome d'hydrogène, et à l'exception du composé de la formule I dans laquelle R₁ représente le radical -CH₃, R₂ et R₅ représentent un atome d'hydrogène, R₃ et R₄ représentent le radical -CH₃, R₆ et R₈ représentent le radical sec.-butyle et R₇ représente le groupe -OH.

Les composés selon l'invention peuvent également se présenter sous forme de sels, lorsqu'ils comportent une fonction acide; il s'agit de sels d'un métal alcalin ou alcalino-terreux ou encore de zinc ou d'une amine organique.

Par radical alkyle ayant de 1 à 6 atomes de carbone, on doit entendre les radicaux méthyle, éthyle, isopropyle, butyle et tertiobutyle.

Par radical alkyle α-substitué ayant de 3 à 15 atomes de carbone on doit entendre un radical isopropyle, 1-méthyl propyle, 1-éthyl propyle, 1-méthyl hexyle, 1-méthyl décyle ou 1-éthyl dodécyle.

Par radical alkyle α,α'-disubstitué ayant de 4 à 12 atomes de carbone on doit notamment entendre un radical tert-butyle, 1,1-diméthylpropyle, 1-méthyl 1-éthyl propyle, 1-méthyl 1-éthyl hexyle ou 1,1-diméthyl décyle.

Par radical monohydroxyalkyle, on doit entendre un radical ayant 2 ou 3 atomes de carbone, notamment un radical 2-hydroxyéthyle, 2-hydroxy- propyle ou 3-hydroxypropyle.

Par radical polyhydroxyalkyle, on doit entendre un radical contenant de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical alkényle on doit entendre un radical ayant de 2 à 6 atomes de carbone, tels que les radicaux vinyle, allyle ou 2-butènyle.

Par radical aryle, on doit entendre un radical phényle éventuellement substitué par au moins un atome d'halogène, un hydroxyle ou une fonction nitro.

Par radical aralkyle on doit entendre le radical benzyle ou phénéthyle éventuellement substitué par au moins un atome d'halogène, un hydroxyle ou une fonction nitro.

Par radical cycloalkyle on doit entendre un radical cyclopentyle ou cyclohexyle.

Par radical cycloalkyle mono ou polycyclique ayant de 5 à 12 atomes de carbone dont le carbone de liaison est trisubstitué on doit entendre le radical 1-méthyl cyclohexyle ou 1-adamantyle.

Lorsque les radicaux r' et r'' pris ensemble forment un hétérocycle, celui-ci est de préférence un radical pipéridino, morpholino, pyrrolidino ou pipérazino, éventuellement substitué en position 4 par un radical alkyle en C₁-C₆ ou mono ou polyhydroxyalkyle tel que défini ci-dessus.

Parmi les composés de formule (I) ci-dessus, on peut notamment citer les suivants:
Acide 4-[3-(1-adamantyl)-4-méthoxybenzoyloxyl] -2-fluorobenzoïque,
Acide 4-[3-(1-adamantyl)-4-méthoxybenzoyloxyl] -2-méthylbenzoïque,
Acide 4-[3-(1-adamantyl)-4-méthoxybenzoyloxy] -2-hydroxybenzoïque,
Acide 4-[3-(1-adamantyl)-4-méthoxybenzoyloxy] -2-méthoxybenzoïque,
Acide 4-[3-(1-adamantyl)-4-méthoxybenzoyloxy] -2-trifluorométhylbenzoïque,
4-[3-(1-adamantyl)-4-méthoxybenzoyloxy] -2-hydroxybenzoate de méthyle,
Acide 4-[5-(1-adamantyl)-2-fluoro-4- méthoxybenzoyloxy]benzoïque,
Acide 4-[5-(1-adamantyl)-2,4-diméthoxybenzoyloxy] benzoïque,
Acide 4-[(3-(1-adamantyl)benzoyloxy]benzoïque,
Acide 4-[3,5-di-tert-butyl-4-hydroxybenzoyloxy] benzoïque,
Acide 4-[3-(1-adamantyl-4-vinylbenzoyloxy]benzoïque,
Acide 4-[3-(1-adamantyl)-4-éthylbenzoyloxy]benzoïque,
Acide 4-[3-(1-adamantyl)-4-butylbenzoyloxy]benzoïque,
Acide 4-[3-(1-adamantyl)-4-allyloxybenzoyloxy] benzoïque,
Acide 4-[3-(1-adamantyl)-4-méthoxybenzoyloxy] benzènesulfonamide,
3-(1-adamantyl)-4-méthoxybenzoate de 4-hydroxyphényle,
3-(1-adamantyl)-4-méthoxybenzoate de phényle,
3-(1-adamantyl)-4-méthoxybenzoate de 4-méthyl phényle,
Acide 4-[3-(1-adamantyl)-4-méthoxybenzoyloxy] -3-hydroxyméthyl benzoïque,
Acide 4-[3-(1-adamantyl)-4-methylthiobenzoyloxy] benzoïque,
Acide 4-[3-(1-adamantyl)-4-méthylsulfone benzoyloxy] benzoïque,
Les composés selon l'invention particulièrement préférés peuvent être représentés par la formule générale (II) 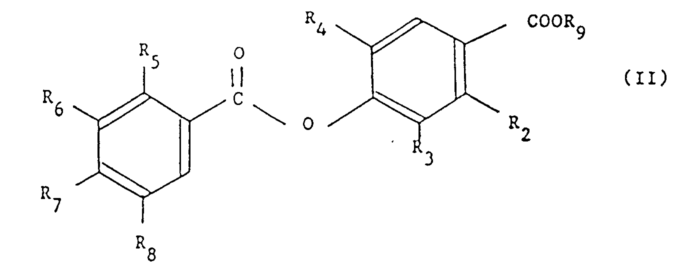 dans laquelle:
(i) soit R₃ à R₉ sont tels que définis ci-dessus pour la formule (I), et
   R₂ représente un radical alkyle ayant de 1 à 6 atomes de carbone, le radical OR₉, un atome de fluor ou le radical -CF₃,
(ii) soit R₂ à R₄ et R₆ à R₉ sont tels que définis ci-dessus pour la formule (I) et R₅ représente un atome de fluor
La présente invention a également pour objet le procédé de préparation des composés de formule (I).
(a) Lorsque R₁ représente un atome d'hydrogène ou le radical -CH₃. les composés selon l'invention peuvent être préparés selon le schéma réactionnel suivant : 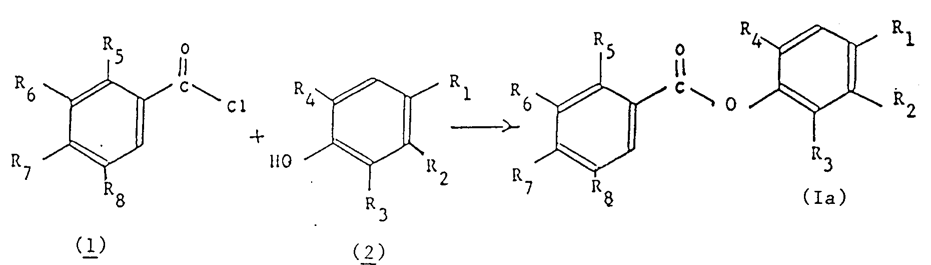 R₁ = H ou CH₃
   L'étape principale de cette préparation consiste à faire réagir en milieu anhydre, dans un solvant organique tel que le tétrahydrofuranne ou le chlorure de méthylène contenant une amine tertiaire (pyridine ou triéthylamine), ou un hydrure alcalin (hydrure de sodium), une forme activée d'un acide benzoïque substitué, par exemple un chlorure d'acide (1), ou un anhydride mixte, sur un composé benzénique porteur d'une fonction hydroxy en para du radical R₁(2), la réaction étant conduite à température ambiante et sous agitation.
(b) Pour les autres significations de R₁, les composés sont préparés en protégeant R₁ par un groupe protecteur du type allylique ou benzylique.

Le passage à la forme libre peut être effectuée, dans le cas d'un groupe protecteur allylique, au moyen d'un catalyseur tel que certains complexes de métaux de transition en présence d'une amine secondaire, et dans le cas d'un groupe protecteur benzylique, par débenzylation en présence d'hydrogène, au moyen d'un catalyseur tel que le palladium sur charbon. Pour R₁= OH le groupe protecteur est préférentiellement benzylique, pour R₁= -CH₂OH ou CH(OH)CH₃, il est preférentiellemnt allylique, pour R₁= COOR₉, il peut être soit allylique soit benzylique. Pour R₁= COOH, les composés de formule Ia peuvent être préparés selon le schéma réactionnel suivant : 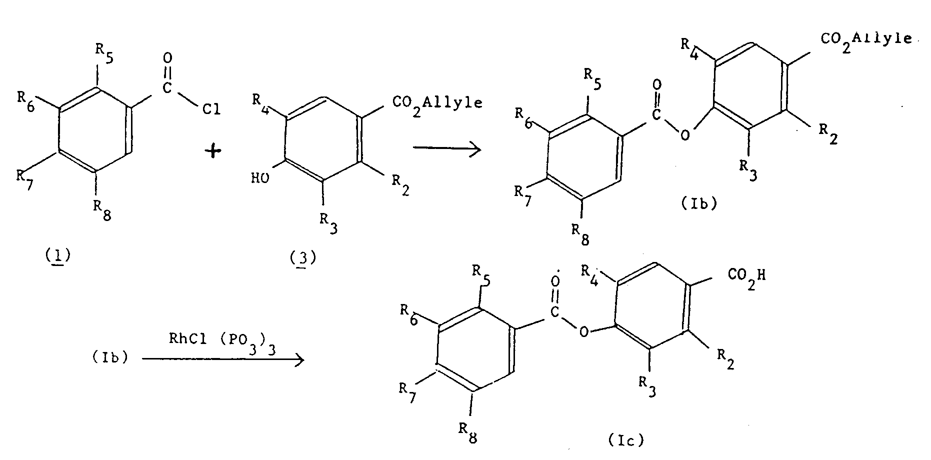 L'action du chlorure d'acide (1) avec un p-hydroxybenzoate d'allyle (3), éventuellement mono ou poly substitué, en présence d'une amine tertiaire telle que la pyridine ou la triéthylamine, conduit aux esters allyliques (Ib). Le passage à l'acide libre peut être effectué au moyen d'un catalyseur tel que certains complexes de métaux de transition par exemple le tris (triphénylphosphine) rhodium (1) chlorure ou le tétrakis (triphénylphosphine) palladium (o) en présence d'une amine secondaire.

Les acides (Ic) ainsi obtenus peuvent être convertis de manière connue en chlorure d'acide correspondant qui, traité par un alcool
(R₉OH) ou une amine 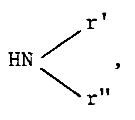 donne l'ester ou l'amide correspondant.

La présente invention a également pour objet à titre de médicament les composés de formule (I) telle que définie ci-dessus.

Les composés selon l'invention présentent une bonne stabilité à la lumière et à l'oxygène.

Ces composés présentent une bonne activité dans le test de différenciation des cellules de tératocarcinome embryonnaire de la souris (cellules F9) (Cancer Research 43, p5268, 1983) et/ou dans le test d'inhibition de l'ornithine décarboxylase après induction par "tape stripping" chez le rat nu (Lab. Animals 21, p233-240, 1987) et/ou dans le test de l'oedème de l'oreille induit par application topique d'acide arachidonique chez la souris (J. Invest. Dermatol. 82, p367-371, 1984). Ces tests montrent les activités des composes respectivement dans les domaines de la différenciation, de la prolifération et de l'inflammation.

Enfin, les nouveaux composés se caractérisent par l'introduction dans la structure chimique, d'une liaison ester hautement sensible à l'action de diverses estérases in vivo, ce qui conduit à l'inactivation rapide des molécules par conversion en fragments biologiquement inactifs.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnés vulgaires, comédoniennes, polymorphes, les acnés nodulo kystiques, conglobata, les acnés séniles, les acnés secondaires telles que l'acné solaire, médicamenteuse, professionnelle ;
2) pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen ;
3) pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriasique, ou encore l'atopie cutanée, telle que l'eczéma, ou l'atopie respiratoire ; les composés peuvent également être utilisés dans certaines affections inflammatoires ne présentant pas de trouble de la kératinisation.
4) pour traiter toutes les proliférations dermiques ou épidermiques qu'elles soient bénignes ou malignes, qu'elles soient d'origine virale telles que les verrues vulgaires, les verrues planes et l'épidermodysplasie verruciforme, les proliférations pouvant également être induites par les ultra-violets notamment dans le cas des épithelioma baso et spino cellulaires ;
5) pour traiter d'autres désordres dermatologiques tels que les dermatoses bulleuses et les maladies du collagène ;
6) pour traiter certains troubles ophtalmologiques, notamment les cornéopathies ;
7) pour lutter contre le vieillissement de la peau, qu'il soit photoinduit ou non ;
8) pour prévenir ou guérir les stigmates de l'atrophie épidermique et/ou dermique induite par les corticostéroïdes locaux ou systémiques, ou toute autre forme d'atrophie cutanée.

La présente invention a donc également pour objet des compositions médicamenteuses contenant au moins un composé de formule (I) tel que défini ci-dessus, ou un de ses sels.

La présente invention a donc aussi pour objet une nouvelle composition médicamenteuse, destinée notamment au traitement des affections susmentionnées, caractérisée par le fait qu'elle comporte, dans un support pharmaceutique acceptable, au moins un composé de formule (I) et/ou un de ses sels.

Les composés selon l'invention sont généralement administrés à une dose journalière d'environ 0,01mg/kg à 100mg/kg de poids corporel.

Comme support des compositions, on peut utiliser tout support conventionnel, le composé actif se trouvant soit à l'état dissous, soit à l'état dispersé dans le véhicule.

L'administration peut être effectuée par voie entérale, parentérale, topique ou oculaire. Par voie entérale, les médicaments peuvent se présenter sous forme de comprimés, de gélules, de dragées, de sirops, de suspensions, de solutions, de poudres, de granulés, d'émulsions ou de nanoparticules. Par voie parentérale, les compositions peuvent se présenter sous forme de solutions, de suspensions ou de nanoparticules pour perfusion ou pour injection.

Par voie topique, les compositions pharmaceutiques à base des composés selon l'invention sont destinées au traitement de la peau et des muqueuses, se présentant sous forme d'onguents, de crèmes, de laits, de pommades, de poudres, de tampons imbibés, de solutions, de gels, de sprays, de lotions ou de suspensions. Elles peuvent également se présenter sous forme de microsphères ou vésicules lipidiques ou polymériques ou de patches polymériques ou d'hydrogels permettant une libération contrôlée.

Ces compositions par voie topique peuvent se présenter soit sous forme anhydre, soit sous forme aqueuse selon l'indication clinique.

Par voie oculaire, ce sont principalement des collyres.

Ces compositions contiennent au moins un composé de formule (I) tel que défini ci-dessus ou un de ses sels, à une concentration de préférence comprise entre 0,0001 et 5% par rapport au poids total de la composition.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels, cette composition se présentant notamment sous forme de lotion, gel, savon ou shampooing.

La concentration en composé de formule (I), dans les compositions cosmétiques est comprise entre 0,0001 et 0,1% en poids et de préférence entre 0,001 et 0,01% en poids.

Les compositions médicamenteuses et cosmétiques selon l'invention peuvent contenir des additifs inertes ou même pharmacodynamiquement ou cosmétiquement actifs et notamment: des agents hydratants comme la thiamorpholinone et ses dérivés ou l'urée; des agents antiséborrhéiques ou antiacnéiques, tels que la S-carboxyméthylcystéine, la S-benzyl-cystéamine, leurs sels et leur dérivés, la tioxolone ou le peroxyde de benzoyle; des antibiotiques comme l'érythromycine et ses esters, la néomycine, les tétracyclines ou les polyméthylène-4,5 isothiazolinones-3; des agents favorisant la repousse des cheveux, comme le "Minoxidil" (2,4-diamino-6- pipéridino-pyrimidine-3-oxyde) et ses dérivés, le Diazoxide (7-chloro 3-méthyl 1,2,4-benzothiadiazine 1,1-dioxyde) et le Phénytoïn (5-5-diphénylimidazolidine 2,4-dione); des agents anti-inflammatoires stéroïdiens et non stéroïdiens; des caroténoïdes et, notamment le β-carotène; des agents anti-psoriasiques tels que l'anthraline et ses dérivés et les acides eicosatétraynoïque-5,8,11,14 et triynoïque-5,8,11, leurs esters et leurs amides.

Les compositions selon l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des anti-oxydants tels que l'α-tocophérol, le butylhydroxyanisole ou le butylhydroxytoluène.

On va maintenant donner, à titre d'illustration et sans aucun caractère limitatif, plusieurs exemples de préparation des composés actifs de formule (I) selon l'invention ainsi que des exemples de compositions les contenant.

### EXEMPLE 1

### Acide 4-[3-(1-adamantyl)-4-méthoxybenzoyloxy] -3-fluorobenzoïque

(a) 3-fluoro-4-hydroxybenzoate d'allyle.
   Dans un ballon, on introduit 5,35g (35mmoles) d'acide 3-fluoro-4-hydroxybenzoïque et 50ml d'alcool allylique. On ajoute 1,5ml d'acide sulfurique concentré et chauffe à 100°C durant douze heures. On évapore à sec, ajoute 200ml d'eau, neutralise avec du bicarbonate de sodium, extrait avec du dichlorométhane. La phase organique est décantée, lavée à l'eau, séchée sur sulfate de magnésium et évaporée. Le résidu obtenu est purifié par chromatographie sur colonne de silice en éluant avec un mélange de dichlorométhane et d'hexane (80-20). On recueille 3,7g (55%) d'ester attendu de point de fusion: 42-43°C.
(b) 4-[3-(1-adamantyl)-4-méthoxybenzoyloxy] -3-fluorobenzoate d'allyle.
   Le chlorure de 3-(1-adamantyl)-4-méthoxybenzoyle préparé à partir de 2,8g (10mmoles) d'acide 3-(1-adamantyl) -4-méthoxybenzoïque, décrit à l'exemple 1(b) de la demande de brevet Européen n°0.232.199 est dissous dans 50ml de tétrahydrofuranne (THF). La solution est ajoutée goutte à goutte sur un mélange de 1,96g (10mmoles) de 3-fluoro-4-hydroxybenzoate d'allyle et de 1,5ml (10mmoles) de triéthylamine dans 50ml de THF. On agite douze heures à température ambiante, verse le milieu réactionnel dans l'eau, et extrait avec de l'éther éthylique. La phase organique est décantée, lavée à l'eau, séchée sur sulfate de magnésium et évaporée. Le résidu est purifié par chromatographie sur colonne de silice en éluant avec un mélange de dichlorométhane et d'hexane (50-50). Après évaporation des solvants, on obtient 3,3g (72%) de l'ester attendu de point de fusion: 123-125°C.
(c) Acide 4-[3-(1-adamantyl)-4-méthoxybenzoyloxy] -3-fluorobenzoïque.
   Dans un ballon et sous courant d'azote, on introduit 108mg (3,6mmoles) d'hydrure de sodium (80% dans l'huile) et 20ml de THF. On ajoute goutte à goutte 546»l (3,6mmoles) de malonate d'éthyle et agite à température ambiante jusqu'à cessation du dégagement gazeux.
   On introduit goutte à goutte cette solution, dans un mélange de 1,65g (3,6mmoles) de l'ester obtenu à l'exemple 1(b)ci-dessus, 208mg de tétrakis (triphénylphosphine) palladium(O) et 30ml de THF. On agite une heure à température ambiante, évapore le milieu réactionnel, triture le résidu obtenu dans 100ml d'éther éthylique et filtre le sel de sodium. On introduit le solide dans 100ml d'eau, acidifie à pH 1 avec de l'acide chlorhydrique concentré, extrait avec de l'éther éthylique, décante la phase organique, sèche sur sulfate de magnésium et évapore. On obtient 1,1g (72%) de l'acide 4-[(3-(1-adamantyl)-4-méthoxybenzoyloxy] -3-fluorobenzoïque de point de fusion: 258-260°C.

### EXEMPLE 2

### Acide 4-[3-(1-adamantyl)-4-méthoxybenzoyloxy] -2-fluorobenzoïque

(a) 2-fluoro-4-hydroxybenzoate d'allyle.
   De manière analogue à l'exemple 1(a), à partir de 8,3g (53mmoles) d'acide 2-fluoro-4-hydroxybenzoïque traités pendant deux heures à 100°C avec 60ml d'alcool allylique et 1,5ml d'acide sulfurique concentré, on obtient 7,2g (69%) d'ester attendu de point de fusion: 80-81°C.
(b) 4-[3-(1-adamantyl)-4-méthoxybenzoyloxy] -2-fluorobenzoate d'allyle.
   A un mélange de 1,96g (10mmoles) de 2-fluoro-4-hydroxybenzoate d'allyle, 1,53ml (11mmoles) de triéthylamine et 25ml de THF, on ajoute goutte à goutte une solution de 3g (10mmoles) de chlorure de 3-(1-adamantyl)-4-méthoxybenzoyle dans 50ml de THF. On agite à température ambiante pendant douze heures, verse dans l'eau le milieu réactionnel et extrait avec de l'éther éthylique. On décante la phase organique, lave à l'eau, sèche sur sulfate de magnésium et évapore. Le résidu obtenu est purifié par chromatographie sur colonne de silice, élué par un mélange de dichlorométhane et d'hexane (50-50). Après évaporation des solvants, on obtient 3,63g (78%) d'ester attendu de point de fusion: 102-104°C.
(c) Acide 4-[3-(1-adamantyl)-4-méthoxybenzoyloxy] -2-fluorobenzoïque.
   Dans un tricol et sous courant d'azote, on introduit 2,32g (5mmoles) d'ester obtenu en 2(b), 145mg de tétrakis (triphénylphosphine) palladium (O) et 25ml de THF anhydre. On ajoute goutte à goutte 4,35ml (50mmoles) de morpholine et agite à température ambiante pendant une heure. On évapore à sec le milieu réactionnel, triture le résidu dans l'éther éthylique et filtre le sel de morpholine formé. On introduit le sel dans 100ml d'eau, acidifie à pH 1 avec de l'acide chlorhydrique concentré, extrait avec de l'éther éthylique, décante la phase organique, lave à l'eau, sèche sur sulfate de magnésium et évapore. Le solide est trituré dans 20ml d'éther éthylique, filtré et séché. On obtient 1,86g (88%) de l'acide attendu de point de fusion: 254-256°C.

### EXEMPLE 3

### Acide 4-[3-(1-adamantyl)-4-méthoxybenzoyloxy] -2-méthylbenzoïque

(a) 4-hydroxy-2-méthylbenzoate d'allyle.
   De manière analogue à l'exemple 1(a), 5,34g (32mmoles) d'acide 4-hydroxy-2-méthyl benzoïque traités à 100°C durant douze heures avec 50ml d'alcool allylique et 1,5ml d'acide sulfurique concentré, donnent 4g (76%) d'ester attendu de point de fusion: 76-77°C.
(b) 4- 3-(1-adamantyl)-4-méthoxybenzoyloxy -2-méthylbenzoate d'allyle.
   Dans un tricol et sous courant d'azote, on introduit 330mg (11mmoles) d'hydrure de sodium (80% dans l'huile) et 20ml de THF. On ajoute goutte à goutte une solution de 1,92g (10mmoles) d'ester préparé à l'exemple 3(a) et agite à température ambiante jusqu'à cessation du dégagement gazeux. On introduit ensuite goutte à goutte une solution de 3g (10mmoles) de chlorure de 3- (1-adamantyl)-4-méthoxybenzoyle dans 30ml de THF et agite à température ambiante pendant six heures. On verse le milieu réactionnel dans l'eau, extrait avec de l'éther éthylique, décante la phase organique, lave à l'eau, sèche sur sulfate de magnésium et évapore. Le résidu obtenu est purifié par chromatographie sur colonne de silice, élué avec un mélange de dichlorométhane et d'hexane (40-60). Après évaporation des solvants, on recueille 2,9g (63%) d'ester attendu de point de fusion: 136-137°C.
(c) Acide 4-[3-(1-adamantyl)-4-méthoxybenzoyloxy] -2-méthylbenzoïque.
   De manière analogue à l'exemple 2(c), à partir de 1,38g (3mmoles) de 4-[3-(1-adamantyl)-4-méthoxybenzoyloxy] -2-méthylbenzoate d'allyle, on obtient 780mg (62%) d'acide 4-[3-(1-adamantyl)-4-méthoxybenzoyloxy]-2-méthylbenzoïque de point de fusion: 236-237°C.

### EXEMPLE 4

### Acide 4-[3-(1-adamantyl)-4-méthoxybenzoyloxy] -2-hydroxybenzoïque

(a) 2,4-dihydroxybenzoate de benzyle.
   A une solution de 3g (0,1mole) d'hydrure de sodium (80% dans l'huile) et 50ml de diméthylformamide (DMF), on ajoute goutte à goutte 15,4g (0,1mole) d'acide 2,4-dihydroxybenzoïque dissous dans 50ml de DMF et agite à température ambiante jusqu'à cessation du dégagement gazeux. On ajoute ensuite 13,1ml (0,1mole) de bromure de benzyle et agite à température ambiante jusqu'à solubilisation du milieu réactionnel. On verse le mélange réactionnel dans l'eau, extrait avec de l'éther éthylique, décante la phase organique, lave à l'eau, sèche sur sulfate de magnésium, évapore. Le résidu est purifié par chromatographie sur colonne de silice en éluant avec du dichlorométhane. On recueille 19,7g (81%) de l'ester attendu de point de fusion: 94-95°C.
(b) 4-[3-(1-adamantyl)-4-méthoxybenzoyloxy] -2-hydroxybenzoate de benzyle.
   De manière analogue à l'exemple 2(b), à partir de 9,8g (40mmoles) de 2,4-dihydroxybenzoate de benzyle, on obtient 16,5g (81%) de 4-[3-(1-adamantyl)-4-méthoxybenzoyloxy] -2-hydroxybenzoate de benzyle de point de fusion: 171-172°C.
(c) Acide 4-[3-(1-adamantyl)-4-méthoxybenzoyloxy] -2-hydroxybenzoïque.
   Dans un réacteur, on introduit 5,1g (10mmoles) d'ester obtenu en 4(b), 1g de palladium sur charbon (5%) et 250ml de dioxanne. On hydrogène à température ambiante et sous une pression de 4 bars, durant une heure. On filtre le catalyseur, évapore le filtrat et triture le solide obtenu dans 100ml d'éther éthylique. Après filtration et séchage, on obtient 3,6g (86%) d'acide 4-[3-(1-adamantyl)-4-méthoxybenzoyloxy]-2-hydroxybenzoïque de point de fusion: 243-244°C.

### EXEMPLE 5

### Acide 4-[3-(1-adamantyl)-4-méthoxybenzoyloxy]-2-méthoxybenzoïque

(a) 4-[3-(1-adamantyl)-4-méthoxybenzoyloxy] -2-méthoxybenzoate de benzyle.
   A une solution de 5,12g (10mmoles) de 4-[3-(1-adamantyl-4-méthoxybenzoyloxy]-2-hydroxybenzoate de benzyle et 200ml de DMF, on ajoute par petites quantités 360mg (12mmoles) d'hydrure de sodium (80% dans l'huile) et agite à température ambiante jusqu'à cessation du dégagement gazeux. On ajoute ensuite 750»l (12mmoles) d'iodure de méthyle et agite quatre heures à température ambiante. On verse le milieu réactionnel dans l'eau extrait avec 300ml d'éther éthylique, décante la phase organique, lave à l'eau, sèche sur sulfate de magnésium, évapore. Le résidu obtenu est purifié par chromatographie sur colonne de silice, élué avec du dichlorométhane. Après évaporation des solvants, on recueille 4,9g (94%) d'ester attendu de point de fusion: 102-103°C.
(b) Acide 4-[3-(1-adamantyl)-4-méthoxybenzoyloxy] -2-méthoxybenzoïque.
   Dans un réacteur, on introduit 4,5g (8,5mmoles) de l'ester benzylique obtenu en 5(a), 450mg de palladium sur charbon (5%) et 120ml de dioxanne. On hydrogène à température ambiante et sous une pression de 4 bars pendant deux heures, filtre le catalyseur et évapore le filtrat. Le solide obtenu est trituré dans 50ml d'éther éthylique, filtré et séché. On recueille 3g (82%) d'acide 4-[3-(1-adamantyl)-4-méthoxybenzoyloxy]-2-méthoxybenzoïque de point de fusion: 199-200°C.

### EXEMPLE 6

### Acide 4-[3-(1-adamantyl)-4-méthoxybenzoyloxy] -3-méthoxybenzoïque

(a) 4-hydroxy-3-méthoxybenzoate d'allyle.
   De manière analogue à l'exemple 1(a), 5g (30mmoles) d'acide 4-hydroxy-3-méthoxybenzoïque traités à 100°C pendant quatre heures avec 50ml d'alcool allylique et 820»l d'acide sulfurique concentré, donnent 4,65g (75%) de l'ester attendu, sous forme d'une huile légèrement jaune.
(b) 4-[3-(1-adamantyl)-4-méthoxybenzoyloxy] -3-méthoxybenzoate d'allyle.
   De manière analogue à l'exemple 2(b), à partir de 2,35g (12mmoles) de 4-hydroxy-3-méthoxybenzoate d'allyle, on obtient 4,12g (77%) d'ester attendu de point de fusion: 146-148°C.
(c) Acide 4-[3-(1-adamantyl)-4-méthoxybenzoyloxy] -3-méthoxybenzoïque.
   De manière analogue à l'exemple 1(c), à partir de 2g (4,2mmoles) de l'ester préparé en 6(b), on obtient 1,35g (74%) d'acide 4-[3-(1-adamantyl)-4-méthoxybenzoyloxy]-3-méthoxybenzoïque de point de fusion: 282-284°C.

### EXEMPLE 7

### Acide 4- 3-(1-adamantyl)-4-méthoxybenzoyloxy 2-trifluorométhylbenzoïque

(a) 4-hydroxy-2-trifluorométhylbenzoate d'allyle.
   De manière analogue à l'exemple 1(a), à partir de 2g (9,7mmoles) d'acide 4-hydroxy-2-trifluorométhylbenzoïque traités à 100°C pendant douze heures avec 20ml d'alcool allylique et 260»l d'acide sulfurique concentré, on obtient 1,4g (59%) de l'ester attendu de point de fusion: 82-83°C.
(b) 4-[3-(1-adamantyl)-4-méthoxybenzoyloxy] -2-trifluorométhylbenzoate d'allyle, on obtient 1,78g (66%) d'ester attendu de point de fusion: 100-102°C.
(c) Acide 4-[3-(1-adamantyl)-4-méthoxybenzoyloxy] -2- trifluorométhylbenzoïque.
   De manière analogue à l'exemple 1(c), à partir de 1,78g (3,46mmoles) de l'ester allylique préparé en 7(b), on obtient 700mg (39%) de l'acide attendu de point de fusion: 228-230°C.

### EXEMPLE 8

### 4-[3-(1-adamantyl)-4-méthoxybenzoyloxy] -2-hydroxybenzoate de méthyle

Dans un ballon, on introduit 1,68g (10mmoles) de 2,4-dihydroxybenzoate de méthyle, 1,55ml (11mmoles) de triéthylamine et 50ml de THF. On ajoute goutte à goutte une solution de 3g (10mmoles) de chlorure de 3-(1-adamantyl)-4-méthoxybenzoyle dans 50ml de THF et agite à température ambiante pendant douze heures. On verse le milieu réactionnel dans l'eau, extrait avec de l'éther éthylique, décante la phase organique, lave à l'eau, sèche sur sulfate de magnésium et évapore. Le résidu obtenu est purifié par chromatographie sur colonne de silice, élué par un mélange de dichlorométhane et d'hexane (50-50). Après évaporation des solvants, on recueille 2,55g (59%) de 4-[3-(1-adamantyl)-4-méthoxybenzoyloxy]-2- hydroxybenzoate de méthyle, de point de fusion: 125-127°C.

### EXEMPLE 9

### Acide 4-[5-(1-adamantyl)-2-fluoro-4-méthoxybenzoyloxy] benzoïque

(a) 5-(1-adamantyl)-2-fluoro-4-hydroxybenzoate d'allyle.
   A une solution de 3g (20mmoles) de 1-adamantanol dans 10ml de n-heptane, on ajoute successivement 63»l d'acide sulfurique concentré et 2,16ml (23mmoles) d'anhydride acétique et agite à température ambiante pendant trois heures. On ajoute ensuite goutte à goutte 540»l (10mmoles) d'acide sulfurique concentré puis une solution de 3,92g (20mmoles) de 2-fluoro-4-hydroxybenzoate d'allyle dans 50ml de dichlorométhane et agite vingt-quatre heures à température ambiante. On évapore à sec le milieu réactionnel, introduit le résidu dans l'eau, neutralise avec du bicarbonate de sodium, filtre le solide et le sèche. Le solide est purifié par chromatographie sur colonne de silice en éluant avec du dichlorométhane. On recueille après évaporation des solvants, 3,86g (58%) de l'ester attendu ayant un point de fusion de: 219-222°C.
(b) 5-(1-adamantyl)-2-fluoro-4-méthoxybenzoate d'allyle.
   Dans un tricol et sous courant d'azote, on introduit 350mg (11,6mmoles) d'hydrure de sodium (80% dans l'huile) et 10ml de DMF. On ajoute goutte à goutte une solution de 3,84g (11,6mmoles) de l'ester préparé à l'exemple 9(a) dans 30ml de DMF et agite à température ambiante jusqu'à cessation du dégagement gazeux. On ajoute goutte à goutte 730»l (11,7mmoles) d'iodure de méthyle et agite à température ambiante pendant six heures. On verse le milieu réactionnel dans l'eau, extrait avec de l'éther éthylique, décante la phase organique, lave à l'eau, sèche sur sulfate de magnésium et évapore. Le résidu obtenu est purifié par chromatographie sur colonne de silice, élué avec un mélange de dichlorométhane et d'hexane (50-50). Après évaporation des solvants, on recueille 2,9g (72%) de 5-(1-adamantyl)-2-fluoro -4-méthoxybenzoate d'allyle de point de fusion: 101-102°C.
(c) Acide 5-(1-adamantyl)-2-fluoro-4-méthoxybenzoïque.
   Une suspension de 2,87g (8,3mmoles) d'ester obtenu en 9(b) dans 100ml de soude méthanolique 2N est chauffée à reflux pendant six heures. On évapore à sec, reprend par l'eau, acidifie à pH 1 avec de l'acide chlorhydrique concentré et extrait avec de l'éther éthylique. On décante la phase organique, lave à l'eau, sèche sur sulfate de magnésium, évapore. On obtient 2,5g (100%) d'acide attendu, qui fond à 282-284°C.
(d) Chlorure de 5-(1-adamantyl)-2-fluoro-4-méthoxybenzoyle.
   2,13g (7mmoles) d'acide 5-(1-adamantyl)-2-fluoro-4-méthoxybenzoïque et 20ml de chlorure de thionyle sont chauffés à reflux jusqu'à cessation du dégagement gazeux. On évapore à sec et obtient 2,26g (100%) du chlorure d'acide brut, qui est utilisé tel quel pour la suite de la synthèse.
(e) 4-[5-(1-adamantyl)-2-fluoro-4-méthoxybenzoyloxy] benzoate d'allyle.
   Dans un ballon, on introduit 1,25g (7mmoles) de 4-hydroxybenzoate d'allyle, 1,1ml (7mmoles) de triéthylamine et 20ml de THF. On ajoute goutte à goutte une solution de 2,26g (7mmoles) du chlorure d'acide préparé en 9(d) dans 60ml de THF et agite à température ambiante durant vingt heures. On verse le milieu réactionnel dans l'eau, extrait avec de l'éther éthylique, décante la phase organique, lave à l'eau, sèche sur sulfate de magnésium et évapore. Le résidu obtenu est purifié par chromatographie sur colonne de silice en éluant avec un mélange de dichlorométhane et d'hexane (70-30). On recueille après évaporation des solvants, 2,4g (74%) de l'ester attendu de point de fusion: 130-131°C.
(f) Acide 4-[5-(1-adamantyl)-2-fluoro-4-méthoxybenzoyloxy]benzoïque.
   De manière analogue à l'exemple 1(c), à partir de 2,33g (5mmoles) de 4-[5-(1-adamantyl)-2-fluoro-4-méthoxybenzoyloxy]benzoate d'allyle, on obtient 1,69g (79%) d'acide 4-[5-(1-adamantyl)-2-fluoro-4-méthoxybenzoyloxy]benzoïque de point de fusion : 268-270°C.

### EXEMPLE 10

### Acide 4-[5-(1-adamantyl)-2,4-diméthoxybenzoyloxy] benzoïque

(a) 5-(1-adamantyl)-2,4-dihydroxybenzoate de méthyle.
   De manière analogue à l'exemple 9(a), à partir de 24,5g (0,146mole) de 2,4-dihydroxybenzoate de méthyle, on obtient 32,5g (74%) de 5-(1-adamantyl)-2,4-dihydroxybenzoate de méthyle de point de fusion: 167-169°C.
(b) 5-(1-adamantyl)-2,4-diméthoxybenzoate de méthyle.
   A une suspension de 1,32g (44mmoles) d'hydrure de sodium (80% dans l'huile) dans 30ml de DMF, on ajoute goutte à goutte une solution de 6g (20mmoles) de l'ester préparé en 10(a) dans 100ml de DMF et agite à température ambiante jusqu'à cessation du dégagement gazeux. On ajoute ensuite 2,75ml (44mmoles) d'iodure de méthyle et agite à température ambiante pendant vingt-quatre heures. On verse le milieu réactionnel dans l'eau, extrait avec de l'éther éthylique, décante la phase organique, lave à l'eau, sèche sur sulfate de magnésium et évapore. Le résidu obtenu est purifié par chromatographie sur colonne de silice, élué avec un mélange de dichlorométhane et d'hexane (90-10). Après évaporation des solvants, on recueille 3,4g (51%) de l'ester attendu de point de fusion: 148-151°C.
(c) Acide 5-(1-adamantyl)-2,4-diméthoxybenzoïque.
   De manière analogue à l'exemple 9(c), à partir de 3,3g (10mmoles) de l'ester méthylique préparé en 10(b), on obtient 3g (94%) de l'acide 5-(1-adamantyl)-2,4-diméthoxybenzoïque de point de fusion: 213-214°C.
(d) Chlorure de 5-(1-adamantyl)-2,4-diméthoxybenzoyle.
   De manière analogue à l'exemple 9(d), à partir de 2,94g (9,3mmoles) d'acide 5-(1-adamantyl)-2,4-diméthoxybenzoïque, on obtient 3,12g (100%) de chlorure d'acide brut, qui est utilisé tel quel pour la suite de la synthèse.
(e) 4-[5-(1-adamantyl)-2,4-diméthoxybenzoyloxy] benzoate d'allyle.
   De manière analogue à l'exemple 9(e), par réaction de 3,12g (9,3mmoles) du chlorure d'acide préparé en 10(d) avec 1,66g (9,3mmoles) de 4-hydroxybenzoate d'allyle, on obtient 1,8g (41%) de l'ester attendu de point de fusion: 120-121°C.
(f) Acide 4-[5-(1-adamantyl)-2,4-diméthoxy benzoyloxy]benzoïque.
   De manière analogue à l'exemple 1(c), à partir de 1,77g (3,71mmoles) de l'ester préparé en 10(e), on obtient 1,16g (72%) d'acide 4-[5-(1-adamantyl)-2,4-diméthoxybenzoyloxy]benzoïque de point de fusion: 236-238°C.

### EXEMPLE 11

### Acide 4-[3-(1-adamantyl)benzoyloxy]benzoïque

(a) 3-(1-adamantyl)-4-hydroxybenzoate de méthyle.
   De manière analogue à l'exemple 9(a), à partir de 22,8g (0,15mole) de 4-hydroxybenzoate de méthyle on obtient 36g (84%) de 3-(1-adamantyl)-4-hydroxybenzoate de méthyle de point de fusion: 183-184°C.
(b) 3-(1-adamantyl)-4-(trifluorométhyl)sulfonyloxybenzoate de méthyle.
   On refroidit à -78°C une solution de 14,4g (50,4mmoles) de 3-(1-adamantyl)-4-hydroxybenzoate de méthyle, 13,6ml (168mmoles) de pyridine, 61mg (0,5mmoles) de 4-diméthylaminopyridine et 100ml de dichlorométhane anhydre et ajoute goutte à goutte 10ml (59,5mmoles) d'anhydride trifluorométhanesulfonique. On laisse remonter la température à 20°C et agite pendant six heures. On verse le milieu réactionnel dans l'eau, acidifie à pH 1 avec de l'acide chlorhydrique concentré et extrait avec du dichlorométhane. On décante la phase organique, lave à l'eau, sèche sur sulfate de magnésium et évapore. Le résidu obtenu est purifié par chromatographie sur colonne de silice en éluant avec de l'hexane. Après évaporation des solvants, on obtient 17,5g (83%) du produit attendu de point de fusion: 90-91°C.
(c) 3-(1-adamantyl)benzoate de méthyle.
   Dans un ballon, on introduit 3g (7,2mmoles) de 3-(1-adamantyl)-4- (trifluorométhyl) sulfonyloxybenzoate de méthyle, 415mg (0,36mmoles) de tétrakis(triphénylphosphine) palladium(O), 3ml (21,5mmoles) de triéthylamine et 15ml de DMF. On ajoute goutte à goutte 547»l (14,4mmoles) d'acide formique et chauffe à 80°C pendant une heure. On verse le milieu réactionnel dans l'eau, extrait avec de l'éther éthylique, décante la phase organique, lave à l'eau, sèche sur sulfate de magnésium et évapore. Le résidu obtenu est purifié par chromatographie sur colonne de silice, élué avec de l'hexane. On recueille 1,78g (90%) de 3-(1-adamantyl)benzoate de méthyle de point de fusion: 147-149°C.
(d) acide 3-(1-adamantyl) benzoïque.
   De manière analogue à l'exemple 9(c), à partir de 3,45g (12,7mmoles) de l'ester préparé en 11(c), on obtient 2,81g (86%) d'acide 3-(1-adamantyl)benzoïque de point de fusion: 247-249°C.
(e) chlorure de 3-(1-adamantyl) benzoyle.
   De manière analogue à l'exemple 9(d), à partir de 1,4g (5,5mmoles) d'acide 3-(1-adamantyl) benzoïque, on obtient 1,5g (100%) du chlorure d'acide brut, utilisé tel quel pour la suite de la synthèse.
(f) 4-[3-(1-adamantyl)benzoyloxy]benzoate d'allyle.
   De manière analogue à l'exemple 9(e), par réaction de 1,5g (5,5mmoles) de chlorure de 3-(1-adamantyl)benzoyle avec 980mg (5,5mmoles) de 4-hydroxybenzoate d'allyle, on obtient 1,5g (68%) de l'ester attendu, sous forme d'une huile incolore.
(g) Acide 4-[3-(1-adamantyl)benzoyloxy]benzoïque.
   De manière analogue à l'exemple 1(c), à partir de 1,45g (3,6mmoles) de 4-[3-(1-adamantyl)benzoyloxy]benzoate d'allyle, on obtient 600mg (40%) d'acide 4-[3-(1-adamantyl) benzoyloxy]benzoïque de point de fusion: 237-239°C.

### EXEMPLE 12

### Acide 4-[3,5-di-tert-butyl-4-hydroxybenzoyloxy] benzoïque

(a) Chlorure de 3,5-di-tert-butyl-4-hydroxybenzoyle.
   1,88g (7,5mmoles) d'acide 3,5-di-tert-butyl-4-hydroxybenzoïque et 15ml de chlorure de thionyle sont chauffés à reflux jusqu'à cessation du dégagement gazeux. On évapore à sec et obtient 2,1g (100%) du chlorure d'acide brut, qui est utilisé tel quel pour la suite de la synthèse.
(b) 4-[3,5-di-tert-butyl-4-hydroxybenzoyloxy] benzoate d'allyle.
   De manière analogue à l'exemple 9(e), par réaction de 2,1g (7,5mmoles) du chlorure d'acide préparé en 12(a) avec 1g (7,5mmoles) de 4-hydroxybenzoate d'allyle, on obtient 1,48g (49%) de l'ester attendu de point de fusion: 114-116°C.
(c) Acide 4-[3,5-di-tert-butyl-4-hydroxybenzoyloxy] benzoïque.
   De manière analogue à l'exemple 2(c), à partir de 1,47g (3,6mmoles) de l'ester préparé en 12(b), on obtient 750mg (57%) d'acide 4-[3,5-di-tert-butyl-4-hydroxybenzoyloxy]benzoïque, qui fond à 223-224°C.

### EXEMPLE 13

### Acide 4-[3-(1-adamantyl)-4-vinylbenzoyloxy]benzoïque

(a) 3-(1-adamantyl)-4-vinylbenzoate de méthyle.
   Dans un ballon et sous argon, on introduit 4,1g (10mmoles) de 3-(1-adamantyl)-4-(trifluorométhyl) sulfonyloxybenzoate de méthyle dans 50ml de DMF et ajoute successivement 3,1ml de vinyltributyletain (10,3mmoles), 1,3g (30mmoles) de chlorure de lithium et 141mg (0,2mmoles) de chlorure de bis-(triphénylphosphine)-palladium(II). On chauffe à 80°C pendant seize heures, verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué par un mélange de dichlorométhane et d'hexane (20-80). On recueille 2,7g (93%) de produit attendu de point de fusion: 86-87°C.
(b) Acide 3-(1-adamantyl)-4-vinylbenzoïque.
   Dans un ballon, on introduit 1,48g (5mmoles) de l'ester précédent, 100ml de soude méthanolique 2N et 250ml de THF. On agite à température ambiante pendant trois heures, évapore à sec, reprend par l'eau, acidifie à pH 1 avec de l'acide chlorhydrique concentré, filtre le solide obtenu et le sèche sous vide en présence de pentoxyde de phosphore. On recueille 1,4g (100%) d'acide attendu de point de fusion: 315-317°C.
(c) Chlorure de 3-(1-adamantyl)-4-vinylbenzoyle.
   Dans un ballon, on introduit 1,4g (5mmoles) d'acide 3-(1-adamantyl)-4-vinylbenzoïque dans 100ml de dichlorométhane et ajoute goutte à goutte 940»l (5mmoles) de dicyclohexylamine. Après obtention d'un milieu réactionnel homogène, on ajoute goutte à goutte 360»l (5mmoles) de chlorure de thionyle et agite à température ambiante pendant une heure. On évapore à sec, reprend par 200ml d'éther éthylique, filtre le sel de dicyclohexylamine et évapore. On obtient 1,5g (100%) de chlorure d'acide brut, qui est utilisé tel quel pour la suite de la synthèse.
(d) 4-[3-(1-adamantyl)-4-vinylbenzoyloxy]benzoate d'allyle.
   De manière analogue à l'exemple 9(e), par réaction de 1,35g (4,5mmoles) de chlorure de 3-(1-adamantyl)-4-vinylbenzoyle avec 800mg (4,5mmoles) de 4-hydroxybenzoate d'allyle, on obtient 1,4g (70%) de l'ester attendu, sous forme d'une huile incolore.
(e) Acide 4-[3-(1-adamantyl)-4-vinylbenzoyloxy] benzoïque.
   De manière analogue à l'exemple 2(c), à partir de 1,2g (2,7mmoles) de 4-[3-(1-adamantyl)-4-vinylbenzoyloxy]benzoate d'allyle, on obtient 750mg (75%) de l'acide 4-[3-(1-adamantyl) -4-vinylbenzoyloxy]benzoïque de point de fusion: 235-236°C.

### EXEMPLE 14

### Acide 4-[3-(1-adamantyl)-4-éthylbenzoyloxy]benzoïque.

(a) 3-(1-adamantyl)-4-éthylbenzoate de méthyle.
   Dans un réacteur, on introduit 3g (10mmoles) de 3-(1-adamantyl)-4- vinylbenzoate de méthyle, 270mg de palladium sur charbon (5%) et 50ml de dioxanne. On hydrogène à température ambiante et sous une pression de 4 bars pendant quatre heures, filtre le catalyseur et évapore le filtrat. On recueille 3g (100%) du produit attendu, sous forme d'une huile incolore.
(b) Acide 3-(1-adamantyl)-4-éthylbenzoïque.
   De manière analogue à l'exemple 13(b), à partir de 2,54g (8,5mmoles) de l'ester précédent, on obtient 2,4g (100%) d'acide 3-(1-adamantyl)-4-éthylbenzoïque.
(c) Chlorure de 3-(1-adamantyl)-4-éthylbenzoyle.
   De manière analogue à l'exemple 9(d), à partir de 2,3g (8,1mmoles) de l'acide précédent, on obtient 2,35g (100%) du chlorure d'acide brut, utilisé tel quel pour la suite de la synthèse.
(d) 4-[3-(1-adamantyl)-4-éthylbenzoyloxy]benzoate d'allyle.
   De manière analogue à l'exemple 9(e), par réaction de 2,35g (8,1mmoles) de chlorure de 3-(1-adamantyl)-4-éthylbenzoyle avec 1,44g (8,1mmoles) de 4-hydroxybenzoate d'allyle, on obtient 2,54g (71%) de l'ester attendu de point de fusion: 111-113°C.
(e) Acide 4-[3-(1-adamantyl)-4-éthylbenzoyloxy] benzoïque.
   De manière analogue à l'exemple 2(c), à partir de 2,5g (5,6mmoles) de l'ester préparé en 14(d), on obtient 1,65g (73%) de l'acide 4- 3-(1-adamantyl)-4-éthylbenzoyloxy benzoïque de point de fusion: 239-241°C.

### EXEMPLE 15

### Acide 4-[3-(1-adamantyl)-4-butylbenzoyloxy]benzoïque

(a) 3-(1-adamantyl)-4-butylbenzoate de méthyle.
   Dans un ballon et sous argon, on introduit 4,1g (10mmoles) de 3-(1-adamantyl)-4-(trifluorométhyl)sulfonyloxybenzoate de méthyle dans 50ml de DMF et ajoute successivement 3,6ml (11mmoles) de tétrabutyletain, 1,3g (30mmoles) de chlorure de lithium et 141mg (0,2mmole) de chlorure de bis-(triphénylphosphine) palladium (II). On chauffe à 80°C durant vingt-quatre heures, verse le milieu réactionnel dans l'eau, extrait avec de l'éther éthylique, décante la phase organique, sèche sur sulfate de magnésium, évapore. Le résidu obtenu est purifié par chromatographie sur colonne de silice en éluant avec un mélange dichlorométhane-hexane (30-70). Après évaporation des solvants, on obtient 2,6g (80%) du produit attendu, sous forme d'une huile légèrement jaune.
(b) Acide 3-(1-adamantyl)-4-butylbenzoïque.
   De manière analogue à l'exemple 13(b), à partir de 2,3g (7mmoles) de l'ester précédent, on obtient 2,1g (100%) d'acide 3-(1-adamantyl)-4-butylbenzoïque de point de fusion: 209-210°C.
(c) Chlorure de 3-(1-adamantyl)-4-butylbenzoyle.
   De manière analogue à l'exemple 9(d), à partir de 2g (6,4mmoles) de l'acide précédent, on obtient 2,1g (100%) du chlorure d'acide brut, utilisé tel quel pour la suite de la synthèse.
(d) 4-[3-(1-adamantyl)-4-butylbenzoyloxy]benzoate de benzyle.
   Le chlorure d'acide précédent, dissout dans 50ml de THF, est ajouté goutte à goutte sur un mélange de 1,46g (6,4mmoles) de 4-hydroxybenzoate de benzyle et de 1ml (7,1mmoles) de triéthylamine dans 50ml de THF. On agite à température ambiante huit heures, verse le milieu réactionnel dans l'eau, extrait avec de l'éther éthylique, décante la phase organique, sèche sur sulfate de magnésium et évapore. Le résidu obtenu est purifié par chromatographie sur colonne de silice en éluant avec un mélange de dichlorométhane et d'hexane (50-50). Après évaporation des solvants, on recueille 2,2g (75%) de l'ester attendu, sous forme d'une huile incolore.
(e) Acide 4-[3-(1-adamantyl)-4-butylbenzoyloxy] benzoïque.
   Dans un réacteur, on introduit 2g (3,8mmoles) de l'ester préparé à l'exemple 15(d), 340mg de palladium sur charbon (5%) et 100ml de dioxanne. On hydrogène à température ambiante et sous une pression de 4 bars pendant quatre heures, filtre le catalyseur, évapore le filtrat. Le résidu obtenu est purifié par chromatographie sur colonne de silice, élué par un mélange de dichlorométhane et d'éther éthylique (70-30). Après évaporation des solvants, on recueille 1,1g (68%) d'acide 4-[3-(1-adamantyl) -4-butylbenzoyloxy]benzoïque de point de fusion: 205-206°C.

### EXEMPLE 16

### Acide 4-[3-(1-adamantyl)-4-allyloxybenzoyloxy]benzoïque

A une solution de 1,17g (3mmoles) d'acide 4-[3-(1-adamantyl)-4- hydroxybenzoyloxy]benzoïque dans 100ml d'un mélange de THF et DMF (50-50), on ajoute par petites quantités 180 mg (6mmoles) d'hydrure de sodium (80% dans l'huile) et agite à température ambiante jusqu'à cessation du dégagement gazeux. On ajoute ensuite 260»l (3mmoles) de bromure d'allyle et agite à température ambiante pendant trois heures. On verse le milieu réactionnel dans l'eau, acidifie à pH 4 avec de l'acide chlorhydrique 1N, extrait avec de l'éther éthylique, décante la phase organique, lave à l'eau, sèche sur sulfate de magnésium et évapore. Le résidu obtenu est purifié par chromatographie sur silice, élué avec de l'éther éthylique. On recueille après évaporation des solvants, 880mg (68%) d'acide 4-[3-(1-adamantyl)-4-allyloxybenzoyloxy]benzoïque de point de fusion: 260-261°C.

### EXEMPLE 17

### Acide 4-[3-(1-adamantyl)-4-méthoxybenzoyloxy] isophtalique

(a) 4-hydroxyisophtalate de benzyle.
   A une solution de 5,5g (30mmoles) d 'acide 4-hydroxyisophtalique dans 200ml de DMF, on ajoute par petites quantités 1,8g (60mmoles) d'hydrure de sodium (80% dans l'huile) et agite jusqu'à cessation du dégagement gazeux. On ajoute ensuite 7,2ml (60mmoles) de bromure de benzyle et agite quatre heures à température ambiante. On verse le milieu réactionnel dans l'eau, extrait avec de l'éther éthylique, décante la phase organique, lave à l'eau, sèche sur sulfate de magnésium et évapore. Le résidu obtenu est purifié par chromatographie sur colonne de silice en éluant avec un mélange de dichlorométhane et d'hexane (30-70). Après évaporation des solvants, on recueille 6,5g (60%) d'ester attendu de point de fusion: 68-70°C.
(b) 4-[3-(1-adamantyl)-4-méthoxybenzoyloxy] isophtalate de benzyle.
   De manière analogue à l'exemple 1(b), par réaction de 5,4g (18mmoles) de chlorure de 3-(1-adamantyl)-4-méthoxybenzoyle avec 6,5g (18mmoles) de 4-hydroxyisophtalate de benzyle, on obtient 7,5g (67%) de 4-[3-(1-adamantyl)-4-méthoxybenzoyloxy]isophtalate de benzyle de point de fusion: 102-104°C.
(c) Acide 4-[3-(1-adamantyl)-4-méthoxybenzoyloxy] isophtalique.
   De manière analogue à l'exemple 15(e), à partir de 3,1g (5mmoles) de l'ester précédent, on obtient 1,2g (55%) d'acide 4-[3-(1-adamantyl)-4-méthoxybenzoyloxy]isophtalique de point de fusion: 172-173°C.

### EXEMPLE 18

### Acide 4-[3-(1-adamantyl)-4-méthoxybenzoyloxy] benzènesulfonamide

Dans un ballon, on introduit 2,86g (10mmoles) d'acide 3-(1-adamantyl)-4-méthoxybenzoïque, 1,73g (10mmoles) de 4-hydroxybenzènesulfonamide et 50ml de THF. On ajoute successivement 2,1g (10mmoles) de 1,3-dicyclohexylcarbodiimide puis 1,22g (10mmoles) de 4-diméthylaminopyridine et agite à température ambiante quatre heures. On verse le milieu réactionnel dans l'eau, extrait à l'éther éthylique, décante la phase organique, lave à l'eau, sèche sur sulfate de magnésium et évapore. Le solide obtenu est purifié par chromatographie sur colonne de silice, élué par un mélange d'éther éthylique et d'hexane (60-40). Après évaporation des solvants, on recueille 2g (46%) de 4-[3-(1-adamantyl) -4-méthoxybenzoyloxy]benzènesulfonamide de point de fusion: 223-224°C.

### EXEMPLE 19

### 3-(1-adamantyl)-4-methoxybenzoate de 4-hydroxyphényle

(a) 3-(1-adamantyl)-4-méthoxybenzoate de 4-benzyloxyphényle.
   Dans un tricol, sous courant d'azote, contenant 663mg (22mmoles) d'hydrure de sodium (80% dans l'huile), on ajoute goutte à goutte 4g (20mmoles) de 4-benzyloxyphénol dans 30 ml de THF anhydre et agite jusqu'à cessation du dégagement gazeux. On introduit ensuite goutte à goutte 6,4g (20mmoles) de chlorure de 3-(1-adamantyl)-4-méthoxybenzoyle, dans 60ml de THF et agite à température ambiante pendant quatre heures. On verse le milieu réactionnel dans l'eau, extrait par du dichlorométhane, lave à l'eau, sèche sur sulfate de magnésium et évapore. Le résidu obtenu est trituré dans l'hexane pour conduire à 9g (96%) de l'ester attendu de point de fusion : 183-185°C.
(b) 3-(1-adamantyl)-4-methoxybenzoate de 4-hydroxyphényle.
   Dans un réacteur, on introduit 4g (8,5mmoles) de l'éther benzylique obtenu à l'exemple 19 (a), 400mg de palladium sur charbon (10%), 70ml de dioxanne, 70ml d'acide acétique et 0,2ml d'acide chlorhydrique (12N). On agite à 50°C sous une pression de 4 bars. Après filtration et évaporation, on reprend le solide avec 400ml de dichlorométhane, et lave la phase organique à l'eau. Après séchage sur sulfate de magnésium, filtration et évaporation, on isole 2,85g (88%) du composé attendu de point de fusion: 236-237°C.

### EXEMPLE 20

### 3-(1-adamantyl)-4-méthoxybenzoate de phényle

De manière analogue à l'exemple 3(b), 0,95g (10mmoles) de phénol dans 10ml de THF, sont traités par 332 mg (11mmoles) d'hydrure de sodium (80% dans l'huile). On introduit ensuite goutte à goutte, une solution de 3,2g (10mmoles) de chlorure de 3-(1-adamantyl)-4-méthoxybenzoyle dans 30ml de THF et laisse agiter à température ambiante pendant 4 heures. On verse le milieu réactionnel dans l'eau, extrait par de l'éther éthylique, lave à l'eau, sèche sur sulfate de magnésium et évapore. Le résidu est chromatographié sur silice, avec un mélange d'hexane et de dichlorométhane (60-40). Après évaporation et précipitation dans l'hexane, on isole 3g (83%) de l'ester attendu de point de fusion : 165-166°C.

### EXEMPLE 21

### 3-(1-adamantyl)-4-méthoxybenzoate de 4-méthylphényle

De manière analogue à l'exemple 3(b), 1,08g (10mmoles) de paracrésol dans 10ml de THF sont traités par 332mg (11mmoles) d'hydrure de sodium (80% dans l'huile). On introduit ensuite goutte à goutte une solution de 3,2g (10mmoles) de chlorure de 3-(1-adamantyl)-4-méthoxybenzoyle dans 30ml de THF et laisse agiter à température ambiante pendant la nuit. On verse le milieu réactionnel dans l'eau et extrait par de l'acétate d'éthyle, lave à l'eau, sèche sur sulfate de magnésium et évapore. le résidu obtenu est recristallisé dans l'acétate d'éthyle pour conduire à 2,91g (77%) de 3-(1-adamantyl)-4-méthoxybenzoate de 4-méthylphényle de point de fusion : 206°C.

### EXEMPLE 22

### Acide 4-[3-(1-adamantyl)-4-méthoxybenzoyloxy] -3-hydroxyméthylbenzoïque

(a) 4-hydroxy-3-hydroxyméthylbenzoate de benzyle.
   Une solution de 27,4g (0,12 moles) de 4-hydroxybenzoate de benzyle, 14,7g (0,12moles) d'acide benzèneboronique, 6g (0,135moles) de paraformaldéhyde et 0,9ml (12mmoles) d'acide propionique dans 600ml de benzène anhydre sont chauffés à reflux durant douze heures en utilisant un Dean-Stark pour séparer l'eau formée. On verse le milieu réactionnel dans l'eau bicarbonatée, extrait avec de l'éther éthylique, décante la phase organique, lave à l'eau, sèche sur sulfate de magnésium et évapore. Le résidu obtenu est purifié par chromatographie sur colonne de silice, élué avec du dichlorométhane. Après évaporation du solvant, on obtient 8,9g (30%) du produit attendu, de point de fusion : 109-110°C.
(b) 3-tert-butyldiméthylsilyloxyméthyl-4-hydroxybenzoate de benzyle.
   A une solution de 2,7g (10,5mmoles) de 4-hydroxy-3-hydroxy méthylbenzoate de benzyle dans 50ml de DMF, on ajoute successivement 1,47ml (10,5mmoles) de triéthylamine et 51mg (0,4mmoles) de 4-N,N-diméthylamino pyridine. On refroidit le milieu réactionnel à 0°C et ajoute goutte à goutte une solution de 1,6g (10,5mmoles) de chlorure de tert-butyldiméthylsilyle dans 20ml de DMF. On agite à température ambiante pendant huit heures, verse le milieu réactionnel dans l'eau, extrait avec de l'éther éthylique, décante la phase organique, sèche sur sulfate de magnésium et évapore. Le résidu obtenu, qui contient deux produits, est purifié par chromatographie sur colonne de silice, en éluant avec un mélange d'hexane et d'éther éthylique (90-10). On obtient ainsi 850mg (23%) de 3-tert-butyldiméthyl silyloxyméthyl-4-hydroxybenzoate de benzyle sous forme d'une huile incolore et 930mg (27%) de 4-tert-butyldiméthylsilyloxy-3-hydroxyméthylbenzoate de benzyle sous forme d'une huile lègèrement jaune.
(c) 4-[3-(1-adamantyl)-4-méthoxybenzoyloxy] -3-tert-butyldiméthyl silyloxyméthylbenzoate de benzyle.
   De manière analogue à l'exemple 1(b), par réaction de 2,2g (7,25mmoles) de chlorure de 3-(1-adamantyl)-4-méthoxybenzoyle avec 2,7g (7,25mmoles) de 3-tert-butyldiméthylsilyloxyméthyl-4-hydroxybenzoate de benzyle, on obtient 3,1g (71%) du produit attendu, sous forme d'huile incolore.
(d) 4-[3-(1-adamantyl)-4-méthoxybenzoyloxy] -3-hydroxyméthyl benzoate de benzyle.
   A une solution de 2,7g (4,5mmoles) de l'ester obtenu à l'exemple 22(c) dans 50ml de THF, on ajoute goutte à goutte 5ml d'une solution molaire de fluorure de tétrabutylammonium dans le THF et agite trente minutes à température ambiante. On verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué avec du dichlorométhane. Après évaporation du solvant, on obtient 700mg (30%) de l'ester attendu sous forme d'une huile opaque.
(e) Acide 4-[3-(1-adamantyl)-4-méthoxybenzoyloxy] -3-hydroxyméthylbenzoïque.
   De manière analogue à l'exemple 15(e), à partir de 700mg (1,3mmoles) de l'ester précédent, on obtient 300mg (55%) d'acide 4-[3-(1-adamantyl)-4-méthoxybenzoyloxy]-3-hydroxyméthylbenzoïque de point de fusion : 214-216°C.

### EXEMPLE 23

### Acide 4-[3-(1-adamantyl)-4-methylthiobenzoyloxy] benzoïque

(a) 3-(1-adamantyl)-4-(diméthylaminothiocarbonyloxy) benzoate de méthyle.
   Dans un ballon, on introduit 2,4 g (0,08 mole) d'hydrure de sodium (80 % dans l'huile) et 250 ml de DMF. Sous courant d'azote, on ajoute goutte à goutte 22,9 g (0,08 mole) de 3-(1-adamantyl)-4-hydroxybenzoate de méthyle et agite jusqu'à cessation du dégagement gazeux. On ajoute ensuite en une seule fois 12,9 g (0,104 mole) de chlorure de diméthylthiocarbamoyle et agite 16 heures à température ambiante. On verse le milieu réactionnel dans l'eau, extrait avec de l'éther éthylique, décante la phase organique, sèche sur sulfate de magnésium, évapore. Le résidu obtenu est purifié par chromatographie sur colonne de silice, élué avec un mélange de dichlorométhane et d'hexane (70-30). Après évaporation des solvants, on recueille 1,8 g (63 %) du produit attendu, de point de fusion : 174°- 175°C.
(b) 3- (1-adamantyl)-4-(diméthylaminocarbonylthio) benzoate de méthyle.
   17,9 g (0,048 mole) de l'ester précédent sont chauffés sous courant d'azote à 300°C pendant 15 minutes. Le résidu obtenu est purifié par chromatographie sur colonne de silice, élué avec un mélange de dichlorométhane et d'hexane (90-10). Après évaporation des solvants, on recueille 14,2 g (80 %) d'ester de point de fusion : 150°-151°c.
(c) Acide 3-(1-adamantyl)-4-mercapto benzoïque.
   Dans un ballon on introduit 14,2 g (38 mmoles) de l'ester précédent, 200 ml de soude méthanolique 2N et chauffe à reflux pendant 4 heures. On évapore à sec le milieu réactionnel, reprend par de l'eau, acidifie à pH = 1 avec de l'acide chlorhydrique concentré, extrait avec de l'éther éthylique, décante la phase organique, sèche sur sulfate de magnésium et évapore. On recueille 10,9 g d'acide brut qui sera utilisé tel quel pour la suite de la synthèse.
(d) 3-(1-adamantyl)-4 méthylthio benzoate de méthyle.
   Dans un ballon, on introduit 2,2 g (73 mmoles) d'hydrure de sodium (80 % dans l'huile) et 50 ml de DMF. On ajoute goutte à goutte une solution de 10,5 g (36,5 mmoles) d'acide 3-(1-adamantyl)-4-mercapto benzoïque dans 100 ml de DMF et agite jusqu'à cessation du dégagement gazeux. On ajoute ensuite 5,7 ml (92 mmoles) d'iodométhane et agite pendant 8 heures. On verse le milieu réactionnel dans l'eau, extrait avec de l'éther éthylique, décante la phase organique, sèche sur sulfate de magnésium et évapore. Le résidu obtenu est purifié par chromatographie sur colonne de silice en éluant avec un mélange de dichlorométhane et d'hexane (40-60). Après évaporation des solvants, on obtient 10 g (87 %) d'ester attendu de point de fusion : 112°-113°C.
(e) Acide 3-(1-adamantyl)-4-méthylthiobenzoïque.
   De manière analogue à l'exemple 1(c), à partir de 2g (6,3 mmoles) de l'ester précédent, on obtient 1,76 g (91 %) d'acide attendu de point de fusion : 264-265°C.
(f) Chlorure de 3-(1-adamantyl)-4-méthylthiobenzoyle.
   1,7 g (5,6 mmoles) d'acide 3-(1-adamantyl)-4-méthylthiobenzoïque et 20ml de chlorure de thionyle sont chauffés à reflux jusqu'à cessation du dégagement gazeux. On évapore à sec et obtient 1,8 g (100 %) de chlorure d'acide brut, qui est utilisé tel quel pour la suite de la synthèse.
(g) 4-[3-(1-adamantyl)-4 méthylthiobenzoyloxy] benzoate de tert-butyle.
   Dans un ballon, on introduit 1 g (5,6 mmoles) de 4-hydroxybenzoate de tert-butyle, 780 »l (5,6 mmoles) de triéthylamine et 50 ml de THF. On ajoute goutte à goutte 1,8 g (5,6 mmoles) du chlorure d'acide préparé en 1(f) dans 50 ml de THF et agite à température ambiante 8 heures. On verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué avec un mélange de dichlorométhane et d'hexane (60-40). Après évaporation des solvants ; on obtient 2,1 g (76 %) d'ester attendu de point de fusion : 133-134°C.
(h) Acide 4-[3-(1-adamantyl)-4-méthylthiobenzoyloxy] benzoïque.
   Dans un ballon, on introduit 1,9 g (3,8 mmoles) de l'ester précédent et 40 ml de tétrachlorure de carbone.
   Sous azote on ajoute goutte à goutte 550 »l (3,8 mmoles) d'iodotriméthylsilane et agite à température ambiante trois heures. On verse le milieu réactionnel dans l'eau, extrait avec de l'éther éthylique, décante la phase organique, sèche sur sulfate de magnésium et évapore. Le solide obtenu est recristallisé dans l'acétate d'éthyle. Après filtration et séchage, on obtient 920 mg (57 %) d'acide 4-[3-(1-adamantyl)-4-méthylthiobenzoyloxy] benzoïque, de point de fusion : 276-277°C.

### EXEMPLE 24

### Acide 4-[3-(1-adamantyl)-4-acétoxybenzoyloxy]benzoïque

(a) 4- 3-(1-adamantyl)-4-acétoxybenzoyloxy benzoate de benzyle.
   Dans un ballon, on introduit 66 mg (2,2 mmoles) d'hydrure de sodium (80 % dans l'huile) et 50 ml de THF, on ajoute goutte à goutte une solution de 964 mg (2 mmoles) de 4-[3-(1-adamantyl)-4-hydroxybenzoyloxy benzoate] de benzyle dans 20 ml de THF et agite jusqu'à cessation du dégagement gazeux. On introduit ensuite 150 »l (2,2 mmoles) de chlorure d'acétyle et agite à température ambiante 8 heures. On verse le milieu réactionnel dans l'eau, extrait avec de l'éther éthylique, décante la phase organique, sèche sur sulfate de magnésium et évapore. Le résidu obtenu est purifié par simple filtration sur silice en éluant avec du dichlorométhane. On recueille après évaporation des solvants 870 mg (83 %) du produit attendu, de point de fusion : 131-132°C.
(b) Acide 4-[3-(1-adamantyl)-4-acétoxybenzoyloxy] benzoïque.
   Dans un réacteur, on introduit 750 mg (1,4 mmoles) de l'ester précédent, 100 mg de palladium sur charbon (10 %) et 20 ml de dioxanne. On hydrogène à température ambiante et sous une pression de 6 bars pendant deux heures, filtre le catalyseur, et évapore le filtrat. Le résidu obtenu est trituré dans le minimum d'éther éthylique, filtré et séché. On obtient 500 mg (81 %) d'acide 4-[3-(1-adamantyl)-4-acétoxybenzoyloxy]benzoïque de point de fusion : 264-265°C.

### EXEMPLE 25

### Acide 4-[3-(1-adamantyl)-4-méthylsulfone benzoyloxy] benzoïque

(a) 4-[3-(1-adamantyl)-4-méthylsulfone benzoyloxy] benzoate de tert-butyle.
   Dans un ballon, on introduit 940 mg (1,9 mmoles) de 4-[3-(1-adamantyl)-4-méthylthiobenzoyloxy]benzoate de tert-butyle et 40 ml de dichlorométhane. On ajoute à 0°C, 700 mg (4 mmoles) d'acide 3-chloroperoxybenzoïque et agite à température ambiante 6 heures. On verse le milieu réactionnel dans l'eau, extrait avec de l'éther éthylique, décante la phase organique, sèche sur sulfate de magnésium et évapore. Le résidu est purifié par chromatographie sur colonne de silice, élué avec de l'éther éthylique. Après évaporation des solvants, on receuille 670 mg (67 %) de sulfone sous forme d'une huile légèrement jaune.
(b) Acide 4-[3-(1-adamantyl)-4-méthylsulfone benzoyloxy]benzoïque.
   De manière analogue à l'exemple 23 (h) à partir de 630 mg (1,2 mmoles) de l'ester précédent, on obtient 190 mg (35 %) d'acide 4-[3-(1-adamantyl)-4-méthylsulfone benzoyloxy]benzoïque de point de fusion : 248-250°C.

### EXEMPLE 26

### Acide 4-[3-(1-adamantyl)-4-(carboxyméthylèneoxy) benzoyloxy]benzoïque.

(a) 4-[3-(1-adamantyl)-4-(benzyloxycarbonylméthylèneoxy) benzoyloxy]benzoate de benzyle.
   De manière analogue à l'exemple 24(a) par réaction de 964 mg (2mmoles) de 4-[3-(1-adamantyl)-4-hydroxybenzoyloxy]benzoate de benzyle avec 350 »l (2,2 mmoles) de 2-bromo acétate de benzyle, on obtient 800 mg (63 %) du produit attendu de point de fusion : 129-130°C.
(b) Acide 4-[3-(1-adamantyl)-4-(carboxyméthylène oxy)benzoyloxy]benzoïque.
   De manière analogue à l'exemple 21 (b) à partir de 750 mg (1,2 mmoles) de l'ester précédent, on obtient 400 mg (75 %) d'acide attendu, de point de fusion : 287-288°C.

### EXEMPLE 27

### Acide 4-[3-(1-adamantyl)-4-(2,3-dihydroxypropyloxy) benzoyloxy]benzoïque.

(a) 3-(1-adamantyl)-4-(2,2-diméthyl-1,3-dioxolane-4-methyloxy) benzoate de méthyle :
   Dans un tricol contenant 10,0 g (34,9 mmoles) d'acide 3-(1-adamantyl)-4-hydroxybenzoïque et 5,31 g (38,4 mmoles) de carbonate de potassium dans 100 ml de diméthylformamide, on ajoute goutte à goutte 12,0 g (41,9 mmoles) de 2,2-diméthyl-1,3-dioxolane -4-méthyltosylate dans 75 ml de diméthylformamide et agite à 100°C pendant 12 heures. Le milieu réactionnel est versé dans de l'eau glacée et extrait par 900 ml d'éther. La phase organique est lavée à l'eau, séchée sur sulfate de magnésium et évaporée. Le résidu est chromatographié sur silice dans l'éluant hexane/acétate d'éthyle (80 : 20) pour conduire après évaporation à 8,82 g (63 %) de 3-(1-adamantyl)-4-(2,2-diméthyl-1,3- dioxolane-4-méthyloxy)benzoate de méthyle de point de fusion : 159°C.
(b) Acide 3-(1-adamantyl)-4-(2,2-diméthyl-1,3-dioxolane-4-methyloxy) benzoïque.
   A une solution de 8,80 g (22 mmoles) de 3-(1-adamantyl -4-(2,2-diméthyl-1,3-dioxolane-4-méthyloxy)benzoate de méthyle dans 200 ml de methanol, on ajoute 16 g (0,4 moles) de soude et chauffe à reflux pendant 3 heures 30 minutes. Le milieu réactionnel est évaporé et repris par 250 ml d'eau, puis acidifié à pH 3. Le précipité est extrait par 1,4 litres d'éther éthylique. La phase organique est lavée à l'eau, séchée sur sulfate de magnésium et évaporée. Après trituration dans l'hexane, on isole 8,32 g (98 %) d'acide 3-(1-adamantyl)-4-(2,2-diméthyl-1,3-dioxolane-4-méthyloxy) benzoïque de point de fusion : 224-225°C.
(c) 4-[3-(1-adamantyl)-4-(2,2-diméthyl-1,3-dioxolane-4-méthyloxy) benzoyloxy]benzoate d'allyle.
   A une solution de 4,15 g (10,75 mmoles) d'acide 3-(1-adamantyl)-4-(2,2-diméthyl-1,3-dioxolane-4-méthyloxy) benzoïque, dans 40 ml de dichlorométhane on ajoute 2,15 ml de dicyclohexylamine (10,8 mmoles), laisse agiter une heure à température ambiante et ajoute goutte à goutte 0,85 ml (11,8 mmoles) de chlorure de thionyle. Après agitation à température ambiante pendant 4 heures, on évapore à sec, reprend dans l'éther éthylique, élimine l'insoluble par filtration et évapore le filtrat. Le chlorure de l'acide 3-(1-adamantyl)-4-(2,2-diméthyl-1,3 dioxolane-4- méthyloxy) benzoïque ainsi obtenu est placé dans 30 ml de THF et ajouté goutte à goutte à une solution sous agitation contenant 1,91 g (10,7 mmoles) de 4-hydroxybenzoate d'allyle, 1,65 ml (11,8 mmoles) de triéthylamine et 13 mg de diméthylaminopyridine dans 30 ml de tétrahydrofurane. Le milieu réactionnel est agité à température ambiante sous azote pendant 20 heures puis est versé dans de l'eau glacée. Cette suspension est extraite par 600 ml d'éther éthylique, puis la phase organique est séchée et évaporée. Le résidu est chromatographié sur silice en éluant par du dichlorométhane pour conduire à 4,24 g (72 %) de 4-[3-(1-adamantyl) -4-(2,2-diméthyl-1,3-dioxolane-4- méthyloxy) benzoyloxy]benzoate d'allyle, de point de fusion : 106-107°C.
(d) 4-[3-(1-adamantyl)-4-(2,3-dihydroxypropyloxy) benzoyloxy]benzoate d'allyle.
   On place 1,09 g (2 mmoles) de 4-[3-(1-adamantyl)-4-(2,2-diméthyl-1,3-dioxolane-4-méthyloxy) benzoyloxy]benzoate d'allyle dans 50 ml de dichlorométhane et ajoute 3,80 g (20 mmoles) d'acide paratoluène sulfonique. La réaction est laissée sous agitation pendant 48 heures à température ambiante puis on évapore à sec et le résidu est repris dans 50 ml de dichlorométhane. La phase organique est neutralisée par lavage avec du NaHCO₃ saturé, puis rincée à l'eau, séchée et évaporée. Le résidu est chromatographié sur silice dans le mélange dichlorométhane/éther éthylique (90 : 10) pour conduire à 900 mg (89 %) de 4-[3-(1-adamantyl)-4-(2,3-dihydroxypropyloxy) benzoyloxy] benzoate d'allyle.
(e) Acide 4-[3-(1-adamantyl)-4-(2,3-dihydroxypropyloxy) benzoyloxy]benzoïque.
   A une solution de 891 mg (1,76 mmoles) de 4-[3-(1-adamantyl)-4-(2,3-dihydroxypropyloxy) benzoyloxy]benzoate d'allyle dans 15 ml de tétrahydrofurane, on ajoute 102 mg (0,088 mmoles) de tetrakis (triphénylphosphine) palladium (0) puis introduit goutte à goutte une suspension constituée de 58 mg (1,93 mmoles) d'hydrure de sodium à 80 % dans l'huile et de 294 »l (1,93 mmoles) de malonate d'éthyle dans 6 ml de tétrahydrofurane. On agite sous azote à température ambiante pendant 1 heure, puis évapore à sec, lave le solide par 50 ml d'éther, le place dans 50 ml d'eau, acidifie à pH 1 et extrait par 100 ml d'éther. La phase organique est rincée à l'eau, séchée sur sulfate de sodium et évaporée. Le résidu est recristallisé dans l'acétate d'éthyle pour conduire à 634 mg (77 %) d'acide 4-[3-(1-adamantyl)-4-(2,3-dihydroxypropyloxy) benzoyloxy]benzoïque, de point de fusion 238-239°C.

### EXEMPLE 28

### Acide 4-[3-(1-adamantyl)-4-(methoxycarbonylmethyloxy)benzoyloxy] benzoïque

(a) 4-[3-(1-adamantyl)-4-tert-butyldimethylsilyloxybenzoyloxy]benzoate de benzyle.
   38,66 g (0,1 mole) d'acide 3-(1-adamantyl) -4- tertbutyldimethylsilyloxy benzoïque sont convertis en chlorure d'acide selon la méthode précédemment décrite, sont mis en solution dans 140 ml de tétrahydrofurane et sont ajoutés goutte à goutte à une suspension contenant 1,66 g (0,055 mmoles) de NaH à 80 % dans l'huile et 1,41 g (0,05 moles) de 4-hydroxybenzoate de benzyle dans 5 ml de tétrahydrofurane. On laisse sous agitation à température ambiante pendant 24 heures puis verse le milieu réactionnel dans de l'eau glacée et extrait avec de l'éther éthylique. La phase organique est lavée à l'eau, séchée sur sulfate de magnésium et évaporée. Le résidu est chromatographié sur silice dans le mélange dichlorométhane/hexane (40 :60) pour conduire à 28 g (47 %) de 4-[3-(1-adamantyl)-4- tertbutyldiméthylsilyloxy]benzoate de benzyle, de point de fusion : 151-152°C.
(b) 4-[3-(1-adamantyl)-4-hydroxybenzoyloxy]benzoate de benzyle.
   Une solution de 5,7 g (9,55 mmoles) de 4-[3-(1-adamantyl)-4-tertbutyldiméthylsilyloxybenzoyloxy]benzoate de benzyle dans 35 ml de tétrahydrofurane est traitée par 10,5 ml de tétrabutylammonium fluoride dans le tétrahydrofurane (1 M). Le milieu réactionnel est agité à température ambiante pendant une heure puis est versé dans de l'eau glacée et extrait par 700 ml d'éther éthylique. La phase organique est lavée par HCl 1N, rincée à l'eau, séchée sur sulfate de magnésium et évaporée. On isole 4,60 g (100 %) de 4-[3-(1-adamantyl)-4-hydroxybenzoyloxy]benzoate de benzyle, de point de fusion : 164-165°C.
(c) 4-[3-(1-adamantyl)-4-(méthoxycarbonylmethyloxy)benzoyloxy] benzoate de benzyle.
   On ajoute goutte à goutte 1,0ml (10,2 mmoles) de bromoacétate de méthyle à une solution de 4,49 g (9,3 mmoles) de 4-[3-(1-adamantyl)-4-hydroxybenzoyloxy]benzoate de benzyle dans 40 ml de diméthylformamide contenant 309 mg (10,2 mmoles) de NaH à 80 % dans l'huile. Le milieu réactionnel est laissé sous agitation à température ambiante pendant la nuit puis est versé dans de l'eau glacée et extrait par 550 ml d'éther éthylique. La phase organique est lavée à l'eau, séchée sur sulfate de magnésium et évaporée. On isole après chromatographie sur silice dans le mélange dichlorométhane/hexane (90 : 10) 3,50 g (68 %) de 4-[3-(1-adamantyl) -4-méthoxycarbonylméthyloxy]benzoate de benzyle de point de fusion : 123-124°C.
(d) Acide 4-[3-(1-adamantyl)-4-(méthoxycarbonyl méthyloxy)benzoyloxy] benzoïque.
   3,46 g (6,24 mmoles) de 4-[3-(1-adamantyl)-4-méthoxycarbonylméthyloxy]benzoate de benzyle dans 50 ml de dioxanne sont hydrogénés sous 7 bars de pression en présence de 346 mg de Pd -C (10 %) durant 2 heures à 40°C. Le milieu réactionnel est filtré sur celite et évaporé pour conduire après recristallisation dans l'acétate d'éthyle à 2,55 g (88 %) d'acide 4-[3-(1-adamantyl)-4-méthoxycarbonylméthyloxy]benzoïque de point de fusion : 252-253°C.

### EXEMPLES DE FORMULATION

### A. VOIE ORALE

| (a) comprimé de 0,2g | |
|---|---|
| Acide 4-[3-(1-adamantyl)-4-méthoxybenzoyloxy] -3-fluoro benzoïque | 0,001g |
| Amidon | 0,114g |
| Phosphate bicalcique | 0,020g |
| Silice | 0,020g |
| Lactose | 0,030g |
| Talc | 0,010g |
| Stéarate de magnésium | 0,005g |

| (b) suspension buvable en ampoules de 5ml | |
|---|---|
| Acide 4-[3-(1-adamantyl)-4-méthoxybenzoyloxy] -2-fluoro benzoïque | 0,001g |
| Glycérine | 0,500g |
| Sorbitol à 70% | 0,500g |
| Saccharinate de sodium | 0,010g |
| Parahydroxybenzoate de méthyle | 0,040g |
| Arôme qs | |
| Eau purifiée qsp | 5ml |

| (c) comprimé de 0,8g | |
|---|---|
| Acide 4-[3-(1-adamantyl)-4-méthoxybenzoyloxy] -2-hydroxybenzoïque | 0,500g |
| Amidon prégélatinisé | 0,100g |
| Cellulose microcristalline | 0,115g |
| Lactose | 0,075g |
| Stéarate de magnésium | 0,010g |

Dans cet exemple (c), l'acide 4-[-3-(1-adamantyl)-4-méthoxybenzoyloxy]-2- hydroxybenzoïque, peut être remplacé par l'acide 4-[5-(1-adamantyl)-2- fluoro-4-méthoxybenzoyloxy] benzoïque.

| (d) suspension buvable en ampoules de 10ml | |
|---|---|
| Acide 4-(3,5-di-tert-butyl-4-hydroxybenzoyloxy) benzoïque | 0,200g |
| Glycérine | 1,000g |
| Sorbitol à 70% | 1,000g |
| Saccharinate de sodium | 0,010g |
| Para hydroxybenzoate de méthyle | 0,080g |
| Arôme qs | |
| Eau purifiée qsp | 10ml |

### B. VOIE TOPIQUE

| (a) onguent | |
|---|---|
| Acide 4-[3-(1-adamantyl)-4-méthoxybenzoyloxy]-3-fluoro benzoïque | 0,020g |
| Myristate d'isopropyle | 81,700g |
| Huile de vaseline fluide | 9,100g |
| Silice vendue par la Société DEGUSSA sous le nom "Aérosil 200" | 9,180g |

| (b) onguent | |
|---|---|
| Acide 4-[3-(1-adamantyl)-4-méthoxybenzoyloxy]-2-fluoro benzoïque | 0,300g |
| Vaseline blanche codex qsp | 100g |

| (c) crème E/H non ionique | |
|---|---|
| Acide 4-(3,5-di-tert-butyl-4-hydroxybenzoyloxy) benzoïque | 0,100g |
| Eucerin anhydre | 39,900g |
| Parahydroxybenzoate de méthyle | 0,075g |
| Parahydroxybenzoate de propyle | 0,075g |
| Eau déminéralisée stérile qsp | 100g |

| (d) lotion | |
|---|---|
| Acide 4-[3-(1-adamantyl)-4-méthoxybenzoyloxy] -2-hydroxybenzoïque | 0,100g |
| PEG 400 | 69,900g |
| Ethanol 95% | 30,000g |

| (e) onguent hydrophobe | |
|---|---|
| Acide 4-[5-(1-adamantyl)-2-fluoro-4-méthoxybenzoyloxy] benzoïque | 0,300g |
| Myristate d'isopropyle | 36,400g |
| Huile de silicone vendue par la Société Rhône Poulenc sous la dénomination "Rhodorsil 47 V 300" | 36,400g |
| Cire d'abeille | 13,600g |
| Huile de silicone vendue par la Société Goldschmidt sous la dénomination "Abil 300.000 cst" qsp | 100g |

| (f) crème H/E non ionique | |
|---|---|
| Acide 4-[3-(1-adamantyl)-4-méthoxybenzoyloxy] -3-fluoro benzoïque | 1,000g |
| Alcool cétylique | 4,000g |
| Monostéarate de glycérol | 2,500g |
| Stéarate de PEG 50 | 2,500g |
| Beurre de karité | 9,200g |
| Propylène glycol | 2,000g |
| Parahydroxybenzoate de méthyle | 0,075g |
| Parahydroxybenzoate de propyle | 0,075g |
| Eau déminéralisée stérile qsp | 100g |

### C. ADMINISTRATION PAR INJECTION

| Ampoule injectable de 3ml | |
|---|---|
| Acide 4-[3-(1-adamantyl)-4-méthoxybenzoyloxy] -2-hydroxybenzoïque micronisé | 0,003g |
| Eau pour préparation injectable qsp | 3 ml |

## Revendications

1. Esters bi-aromatiques, caractérisés par le fait qu'ils sont choisis parmi les suivants :
Acide 4-[3-(1-adamantyl)-4-méthoxybenzoyloxy]- 3-fluorobenzoïque,
Acide 4-[3-(1-adamantyl)-4-méthoxybenzoyloxy]- 3-méthoxybenzoïque,
Acide 4-[3-(1-adamantyl)-4-méthoxybenzoyloxy]-isophtalique,
Acide 4-[3-(1-adamantyl)-4-acétoxybenzoyloxy]-benzoïque,
Acide 4-[3-(1-adamantyl)-4-(carboxyméthylèneoxy) benzoyloxy] benzoïque,
Acide 4-[3-(1-adamantyl)-4-(2,3-dihydroxy-propyloxy) benzoyloxy] benzoïque,
Acide 4-[3-(1-adamantyl)-4-(méthoxycarbonylméthyloxy) benzoyloxy] benzoïque, et ceux répondant à la formule générale suivante : 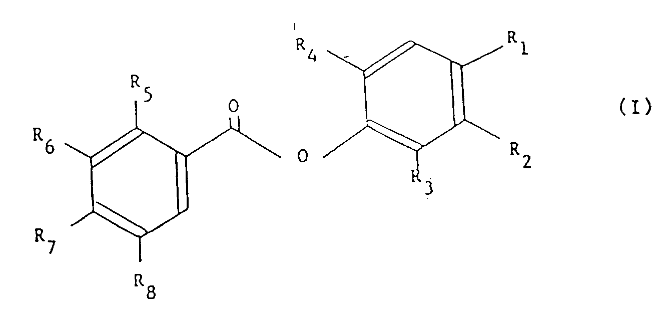
dans laquelle:
R₁ représente le groupe OH, le radical -CH₃, -CH₂OH, -CH(OH)CH₃, -COOR₉, 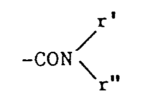 ou SO₂R₁₀,
R₉ représente un atome d'hydrogène, un radical alkyle ayant de 1 à 6 atomes de carbone ou un radical mono ou polyhydroxyalkyle,
R₁₀ représentant le groupe OH, un radical alkyle ayant de 1 à 6 atomes de carbone ou le radical 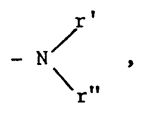 r' et r'' représentant un atome d'hydrogène, un radical alkyle ayant de 1 à 6 atomes de carbone, aryle, aralkyle, mono ou polyhydroxyalkyle, ou r' et r'' pris ensemble forment un hétérocycle,
R₂ représente un atome d'hydrogène, un radical alkyle ayant de 1 à 6 atomes de carbone, le radical OR₉ , un atome de fluor ou le radical -CF₃,
R₃ , R₄ et R₅ représentent un atome d'hydrogène, un atome de fluor, le groupe OH, le radical -CH₃, -OCH₃, -CF₃,-COOH ou -CH₂OH,
R₆ et R₈ représentent un atome d'hydrogène, un radical alkyle α-substitué ayant de 3 à 15 atomes de carbone, un radical alkyle α-α' disubstitué ayant de 4 à 12 atomes de carbone, un radical cycloalkyle ayant de 3 à 12 atomes de carbone, un radical cycloalkyle mono ou polycyclique ayant de 5 à 12 atomes de carbone dont le carbone de liaison est trisubstitué, le radical -SR₁₁, -SO₂R₁₁ ou -SOR₁₁.
R₁₁ représentant un radical alkyle ayant de 1 à 6 atomes de carbone ou un radical cycloalkyle, R₆ et R₈ ne pouvant pas simultanément représenter un atome d'hydrogène,
R₇ représente un atome d'hydrogène, un radical alkyle ayant de 1 à 6 atomes de carbone, un radical alkényle, un radical alkényloxy, le radical OR₁₂, SR₁₃, SOR₁₄ ou SO₂R₁₄,
R₁₂ représentant un atome d'hydrogène, un radical alkyle ayant de 1 à 6 atomes de carbone, un radical alkényle, un radical mono ou polyhydroxy alkyle ou le radical -(CH₂)ₙ-COR₁₅,
n étant 0,1 ou 2 et R₁₅ représentant un atome d'hydrogène, le groupe OH, un radical alkyle ayant de 1 à 6 atomes de carbone ou un radical alkoxy ayant de 1 à 6 atomes de carbone.
R₁₃ représentant un atome d'hydrogène, un radical alkyle ayant de 1 à 6 atomes de carbone ou un radical aralkyle,
R₁₄ représentant le groupe OH, un radical alkyle ayant de 1 à 6 atomes de carbone ou un radical aralkyle,
sous réserve que lorsque R₁ représente -CH₂OH, -CH(OH)CH₃, -COOR₉ ou 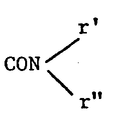 et R₂ représente un atome d'hydrogène alors :
(i) soit R₃ et R₄ sont différents d'un atome d'hydrogène ou du radical -CH₃ ,
(ii) soit R₇ est différent du radical OR₁₂ et R₆ ou R₈ est un radical cycloalkyle ayant plus de 7 atomes de carbone,
(iii) soit R₇ représente le radical OR₁₂ , mais R₆ et R₈ sont alors différents d'un atome d'hydrogène,
(iv) soit R₇ représente le radical OR₁₂ , mais alors R₅ est différent d'un atome d'hydrogène, et à l'exception du composé de la formule I dans laquelle R₁ représente le radical -CH₃, R₂ et R₅ représentent un atome d'hydrogène, R₃ et R₄ représentent le radical -CH₃, R₆ et R₈ représentent le radical sec.-butyle et R₇ représente le groupe -OH.

2. Composés selon la revendication 1, caractérisés par le fait qu'ils se présentent sous forme de sels d'un métal alcalin ou alcalino-terreux ou encore de zinc ou d'une amine organique.

3. Composés selon la revendication 1, caractérisés par le fait que le radical alkyle inférieur a de 1 à 6 atomes de carbone et est pris dans le groupe constitué par les radicaux méthyle, éthyle, isopropyle, butyle et tertiobutyle.

4. Composés selon la revendication 1, caractérisés par le fait que le radical alkyle α-substitué est pris dans le groupe constitué par un radical isopropyle, 1-méthyl propyle, 1-éthyl propyle, 1-méthyl hexyle, 1-méthyl décyle et 1-éthyl dodécyle.

5. Composés selon la revendication 1, caractérisés par le fait que le radical alkyle α,α'-disubstitué est pris dans le groupe constitué par: un radical tert-butyle, 1,1-diméthyl propyle, 1-méthyl 1-éthyl propyle, 1-méthyl 1-éthyl hexyle et 1,1-diméthyl décyle.

6. Composés selon la revendication 1, caractérisés par le fait que le radical monohydroxyalkyle est un radical 2-hydroxyéthyle, 2-hydroxypropyle ou 3-hydroxypropyle.

7. Composés selon la revendication 1, caractérisés par le fait que le radical polyhydroxyalkyle comporte de 3 à 6 atomes de carbone et de 2 à 5 groupes hydroxyles et est pris dans le groupe constitué par le radical 2,3-dihydroxypropyle, 2,3,4-trihydroxybutyle, 2,3,4,5-tétrahydroxy pentyle et le reste du pentaérythritol.

8. Composés selon la revendication 1, caractérisés par le fait que le radical alkényle ayant de 2 à 6 atomes de carbone est le radical vinyle, allyle ou 2-butène-yle.

9. Composés selon la revendication 1, caractérisés par le fait que le radical aryle est un radical phényle éventuellement substitué par au moins un atome d'halogène, un hydroxyle ou une fonction nitro.

10. Composés selon la revendication 1, caractérisés par le fait que le radical aralkyle est le radical benzyle ou le radical phénéthyle éventuellement substitué par au moins un atome d'halogène, un hydroxyle ou une fonction nitro.

11. Composés selon la revendication 1, caractérisés par le fait que le radical cycloalkyle mono ou polycyclique ayant de 5 à 12 atomes de carbone dont le carbone de liaison est trisubstitué est le radical 1-méthyl cyclohexyle ou 1-adamantyle.

12. Composés selon la revendication 1, caractérisés par le fait que les radicaux r' et r'' pris ensemble forment un hétérocycle pris dans le groupe constitué par un radical pipéridino, morpholino, pyrrolidino ou pipérazino éventuellement substitué en position 4 par un radical alkyle en C₁-C₆ ou mono ou polyhydroxyalkyle.

13. Composés selon l'une quelconque des revendications précédentes, caractérisés par le fait qu'ils sont pris dans le groupe constitué par:
Acide 4-[3-(1-adamantyl)-4-méthoxybenzoyloxyl]-2-fluorobenzoïque,
Acide 4-[3-(1-adamantyl)-4-méthoxybenzoyloxyl]-2-méthylbenzoïque,
Acide 4-[3-(1-adamantyl)-4-méthoxybenzoyloxy]-2-hydroxybenzoïque,
Acide 4-[3-(1-adamantyl)-4-méthoxybenzoyloxy]-2-méthoxybenzoïque,
Acide 4-[3-(1-adamantyl)-4-méthoxybenzoyloxy]-2-trifluorométhylbenzoïque,
4-[3-(1-adamantyl)-4-méthoxybenzoyloxy]-2-hydroxybenzoate de méthyle,
Acide 4-[5-(1-adamantyl)-2-fluoro-4-méthoxybenzoyloxy]benzoïque,
Acide 4-[5-(1-adamantyl)-2,4-diméthoxybenzoyloxy] benzoïque,
Acide 4-[(3-(1-adamantyl)benzoyloxy]benzoïque,
Acide 4-[3,5-di-tert-butyl-4-hydroxybenzoyloxy] benzoïque,
Acide 4-[3-(1-adamantyl-4-vinylbenzoyloxy]benzoïque,
Acide 4-[3-(1-adamantyl)-4-éthylbenzoyloxy] benzoïque,
Acide 4-[3-(1-adamantyl)-4-butylbenzoyloxy] benzoïque,
Acide 4-[3-(1-adamantyl)-4-allyloxybenzoyloxy] benzoïque,
Acide 4-[3-(1-adamantyl)-4-méthoxybenzoyloxy] benzènesulfonamide,
3-(1-adamantyl)-4-méthoxybenzoate de 4-hydroxyphényle,
3-(1-adamantyl)-4-méthoxybenzoate de phényle,
3-(1-adamantyl)-4-méthoxybenzoate de 4-méthyl phényle,
Acide 4-[3-(1-adamantyl)-4-méthoxybenzoyloxy] -3-hydroxyméthyl benzoïque,
Acide 4-[3-(1-adamantyl)-4-methylthiobenzoyloxy] benzoïque,
Acide 4-[3-(1-adamantyl)-4-méthylsulfone benzoyloxy] benzoïque,

14. Composés selon l'une quelconque des revendications précédentes, caractérisés par le fait qu'ils répondent de préférence à la formule générale suivante: 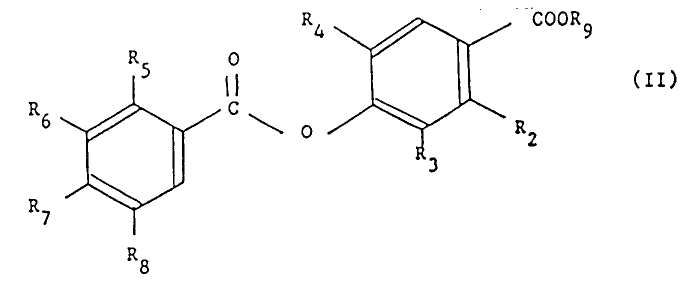 dans laquelle:
(i) soit R₃ à R₉ sont tels que définis à la revendication 1, et
R₂ représente un radical alkyle ayant de 1 à 6 atomes de carbone, le radical OR₉ , un atome de fluor ou le radical -CF₃.
(ii) soit R₂ à R₄ et R₆ à R₉ sont tels que définis à la revendication 1 et R₅ représente un atome de fluor.

15. Procédé de préparation des composés de formule (I) selon l'une quelconque des revendications 1 à 14, dans lesquels R₁ = H ou -CH₃, caractérisé par le fait qu'il consiste à faire réagir, dans un solvant organique, en présence d'une amine tertiaire, ou d'un hydrure alcalin, une forme activée d'un acide benzoïque substitué notamment un chlorure d'acide de formule: 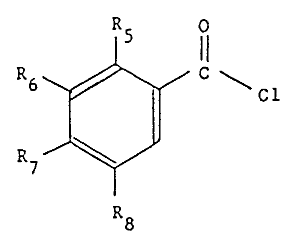 sur un dérivé p-hydroxy benzénique de formule: 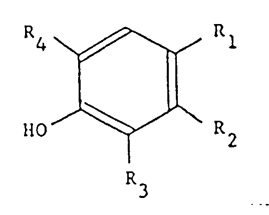 dans lesquelles:
R₁ représente un atome d'hydrogène ou le radical -CH₃, et R₂ à R₈ ont les mêmes significations que celles données à la revendication 1, la réaction étant conduite à température ambiante et sous agitation.

16. Procédé de préparation des composés de formule (I) selon l'une quelconque des revendications 1 à 14, dans lesquels R₁ = -COOR₉ ou 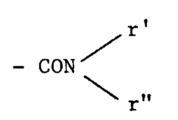 caractérisé par le fait qu'il consiste à faire réagir, en présence d'une amine tertiaire, un chlorure d'acide de formule: 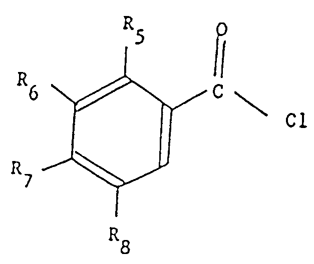 sur un p-hydroxybenzoate d'allyle de formule: 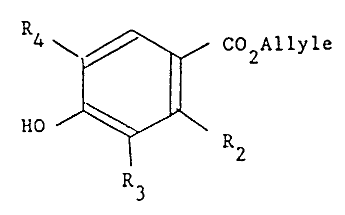 dans lesquelles:
R₂ à R₈ ont les mêmes significations que celles données à la revendication 1, que l'on transforme l'ester allylique obtenu en acide correspondant en présence, comme catalyseur, d'un complexe de métaux de transition, et que l'on procède, si nécessaire, à la formation du chlorure d'acide correspondant et à la transformation subséquente dudit chlorure soit en ester par action d'un alcool (R₉OH) soit en amide par action d'une amine

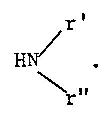

17. Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 14.

18. Composition selon la revendication 17, caractérisée par le fait qu'elle contient de 0,0001 à environ 5% en poids d'un composé de formule (I).

19. Utilisation d'un composé selon l'une quelconque des revendications 1 à 14, pour la préparation d'une composition pharmaceutique destinée au traitement des affections dermatologiques, rhumatismales, respiratoires ainsi qu'ophtalmologiques.

20. Composition cosmétique pour l'hygiène corporelle et capillaire, caractérisée par le fait qu'elle contient, dans un véhicule cosmétique approprié, au moins un composé de formule (I) selon l'une quelconque des revendications 1 à 14.

21. Composition cosmétique selon la revendication 20, caractérisée par le fait qu'elle contient le composé de formule (I) à une concentration comprise entre 0,0001 et 0,1% et de préférence entre 0,001 et 0,01% en poids.

## Claims

1. Biaromatic esters, characterized in that they are chosen from the following:
4-[3-(1-adamantyl)-4-methoxybenzoyloxy]-3-fluorobenzoic acid,
4-[3-(1-adamantyl)-4-methoxybenzoyloxy]-3-methoxybenzoic acid,
4-[3-(1-adamantyl)-4-methoxybenzoyloxy]isophthalic acid,
4-[3-(1-adamantyl)-4-acetoxybenzoyloxy]benzoic acid,
4-[3-(1-adamantyl)-4-(carboxymethyloxy)benzoyloxy]benzoic acid,
4-[3-(1-adamantyl)-4-(2,3-dihydroxypropyloxy)benzoyloxy]benzoic acid,
4-[3-(1-adamantyl)-4-(methoxycarbonylmethyloxy)benzoyloxy]benzoic acid, and those corresponding to the following general formula: 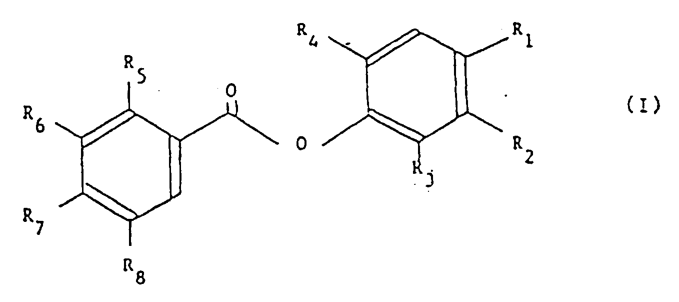 in which:
R₁ represents the OH group or the radical
-CH₃, -CH₂OH, -CH(OH)CH₃, -COOR₉, 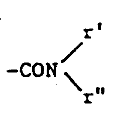 or SO₂R₁₀,
R₉ represents a hydrogen atom, an alkyl radical having from 1 to 6 carbon atoms or a mono- or polyhydroxyalkyl radical,
R₁₀ representing the OH group, an alkyl radical having from 1 to 6 carbon atoms or the radical 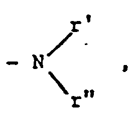 r' and r'' representing a hydrogen atom, an alkyl radical having from 1 to 6 carbon atoms, an aryl radical, an aralkyl radical or a mono- or polyhydroxyalkyl radical, or r' and r'', taken together, form a heterocycle,
R₂ represents a hydrogen atom, an alkyl radical having from 1 to 6 carbon atoms, the radical OR₉, a fluorine atom or the -CF₃ radical,
R₃, R₄ and R₅ represent a hydrogen atom, a fluorine atom, the OH group, or the -CH₃, -OCH₃, -CF₃, -COOH or -CH₂OH radical,
R₆ and R₈ represent a hydrogen atom, an α-substituted alkyl radical having from 3 to 15 carbon atoms, an α,α'-disubstituted alkyl radical having from 4 to 12 carbon atoms, a cycloalkyl radical having from 3 to 12 carbon atoms, a mono- or polycyclic cycloalkyl radical having from 5 to 12 carbon atoms, the bonding carbon of which is trisubstituted, or the radical -SR₁₁, -SO₂R₁₁ or -SOR₁₁,
R₁₁ representing an alkyl radical having from 1 to 6 carbon atoms or a cycloalkyl radical, it not being possible for R₆ and R₈ simultaneously to represent a hydrogen atom,
R₇ represents a hydrogen atom, an alkyl radical having from 1 to 6 carbon atoms, an alkenyl radical, an alkenyloxy radical or the radical OR₁₂, SR₁₃, SOR₁₄ or SO₂R₁₄,
R₁₂ representing a hydrogen atom, an alkyl radical having from 1 to 6 carbon atoms, an alkenyl radical, a mono- or polyhydroxyalkyl radical or the radical -(CH₂)ₙ-COR₁₅,
n being 0, 1 or 2 and R₁₅ representing a hydrogen atom, the OH group, an alkyl radical having from 1 to 6 carbon atoms or an alkoxy radical having from 1 to 6 carbon atoms,
R₁₃ representing a hydrogen atom, an alkyl radical having from 1 to 6 carbon atoms or an aralkyl radical,
R₁₄ representing the OH group, an alkyl radical having from 1 to 6 carbon atoms or an aralkyl radical, with the proviso that when R₁ represents -CH₂OH, -CH(OH)CH₃, -COOR₉ or 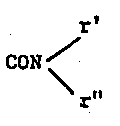 and R₂ represents a hydrogen atom, then:
(i) either R₃ and R₄ are other than a hydrogen atom or the -CH₃ radical,
(ii) or R₇ is other than the radical OR₁₂ and R₆ or R₈ is a cycloalkyl radical having more than 7 carbon atoms,
(iii) or R₇ represents the radical OR₁₂, but R₆ and R₈ are then other than a hydrogen atom,
(iv) or R₇ represents the radical OR₁₂, but then R₅ is other than a hydrogen atom,
and with the exception of the compound of the formula (I) in which R₁ represents the -CH₃ radical, R₂ and R₅ represent a hydrogen atom, R₃ and R₄ represent the -CH₃ radical, R₆ and R₈ represent the sec-butyl radical and R₇ represents the -OH group.

2. Compounds according to Claim 1, characterized in that they are provided in the form of salts of an alkali metal or alkaline-earth metal or alternatively of zinc or of an organic amine.

3. Compounds according to Claim 1, characterized in that the lower alkyl radical has from 1 to 6 carbon atoms and is taken from the group consisting of the methyl, ethyl, isopropyl, butyl and tert-butyl radicals.

4. Compounds according to Claim 1, characterized in that the α-substituted alkyl radical is taken from the group consisting of the isopropyl, 1-methylpropyl, 1-ethylpropyl, 1-methylhexyl, 1-methyldecyl and 1-ethyldodecyl radicals.

5. Compounds according to Claim 1, characterized in that the α,α'-disubstituted alkyl radical is taken from the group consisting of: the tert-butyl, 1,1-dimethylpropyl, 1-methyl-1-ethylpropyl, 1-methyl-1-ethylhexyl and 1,1-dimethyldecyl radicals.

6. Compounds according to Claim 1, characterized in that the monohydroxyalkyl radical is a 2-hydroxyethyl, 2-hydroxypropyl or 3-hydroxypropyl radical.

7. Compounds according to Claim 1, characterized in that the polyhydroxyalkyl radical contains from 3 to 6 carbon atoms and from 2 to 5 hydroxyl groups and is taken from the group consisting of the 2,3-dihydroxypropyl, 2,3,4-trihydroxybutyl and 2,3,4,5-tetrahydroxypentyl radicals and the pentaerythritol residue.

8. Compounds according to Claim 1, characterized in that the alkenyl radical having from 2 to 6 carbon atoms is the vinyl, allyl or 2-butenyl radical.

9. Compounds according to Claim 1, characterized in that the aryl radical is a phenyl radical optionally substituted by at least one halogen atom, a hydroxyl or a nitro functional group.

10. Compounds according to Claim 1, characterized in that the aralkyl radical is the benzyl radical or the phenethyl radical optionally substituted by at least one halogen atom, a hydroxyl or a nitro functional group.

11. Compounds according to Claim 1, characterized in that the mono- or polycyclic cycloalkyl radical having from 5 to 12 carbon atoms, the bonding carbon of which is trisubstituted, is the 1-methylcyclohexyl or 1-adamantyl radical.

12. Compounds according to Claim 1, characterized in that the radicals r' and r'', taken together, form a heterocycle taken from the group consisting of a piperidino radical, a morpholino radical, a pyrrolidino radical or a piperazino radical optionally substituted in the 4-position by a mono- or polyhydroxyalkyl or C₁-C₆ alkyl radical.

13. Compounds according to any one of the preceding claims, characterized in that they are taken from the group consisting of:
4-[3-(1-adamantyl)-4-methoxybenzoyloxy]-2-fluorobenzoic acid,
4-[3-(1-adamantyl)-4-methoxybenzoyloxy]-2-methylbenzoic acid,
4-[3-(1-adamantyl)-4-methoxybenzoyloxy]-2-hydroxybenzoic acid,
4-[3-(1-adamantyl)-4-methoxybenzoyloxy]-2-methoxybenzoic acid,
4-[3-(1-adamantyl)-4-methoxybenzoyloxy]-2-trifluoromethylbenzoic acid,
methyl 4-[3-(1-adamantyl)-4-methoxybenzoyloxy]-2-hydroxybenzoate,
4-[5-(1-adamantyl)-2-fluoro-4-methoxybenzoyloxy]benzoic acid,
4-[5-(1-adamantyl)-2,4-dimethoxybenzoyloxy]benzoic acid,
4-[3-(1-adamantyl)benzoyloxy]benzoic acid,
4-[3,5-di-tert-butyl-4-hydroxybenzoyloxy]benzoic acid,
4-[3-(1-adamantyl)-4-vinylbenzoyloxy]benzoic acid,
4-[3-(1-adamantyl)-4-ethylbenzoyloxy]benzoic acid,
4-[3-(1-adamantyl)-4-butylbenzoyloxy]benzoic acid,
4-[3-(1-adamantyl)-4-allyloxybenzoyloxy]benzoic acid,
4-[3-(1-adamantyl)-4-methoxybenzoyloxy]benzenesulphonamide,
4-hydroxyphenyl 3-(1-adamantyl)-4-methoxybenzoate,
phenyl 3-(1-adamantyl)-4-methoxybenzoate,
4-methylphenyl 3-(1-adamantyl)-4-methoxybenzoate,
4-[3-(1-adamantyl)-4-methoxybenzoyloxy]-3-(hydroxymethyl)benzoic acid,
4-[3-(1-adamantyl)-4-(methylthio)benzoyloxy]benzoic acid,
4-[3-(1-adamantyl)-4-(methylsulphonyl)benzoyloxy]benzoic acid.

14. Compounds according to any one of the preceding claims, characterized in that they preferably correspond to the following general formula: 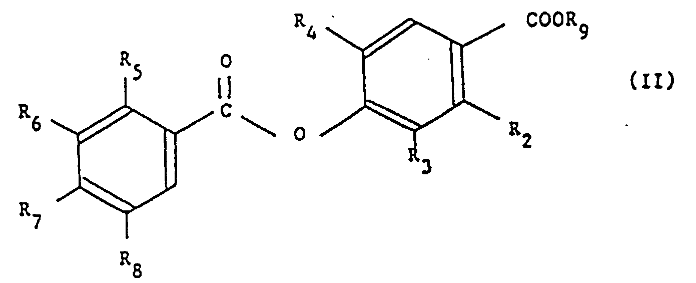 in which
(i) either R₃ to R₉ are as defined in Claim 1, and
R₂ represents an alkyl radical having from 1 to 6 carbon atoms, the radical OR₉, a fluorine atom or the -CF₃ radical,
(ii) or R₂ to R₄ and R₆ to R₉ are as defined in Claim 1 and R₅ represents a fluorine atom.

15. Process for the preparation of the compounds of formula (I) according to any one of Claims 1 to 14, in which R₁ = H or -CH₃, characterized in that it consists in reacting, in an organic solvent, in the presence of a tertiary amine or of an alkali metal hydride, an activated form of a substituted benzoic acid, especially an acid chloride of formula: 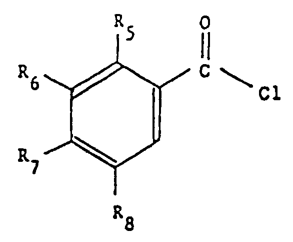 with a p-hydroxybenzene derivative of formula: 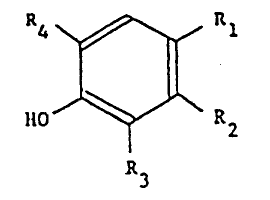 in which:
R₁ represents a hydrogen atom or the -CH₃ radical, and R₂ to R₈ have the same meanings as those given in Claim 1, the reaction being carried out at room temperature and with stirring.

16. Process for the preparation of the compounds of formula (I) according to any one of Claims 1 to 14, in which R₁ = -COOR₉ or 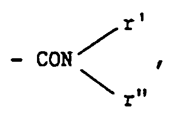 characterized in that it consists in reacting, in the presence of a tertiary amine, an acid chloride of formula: 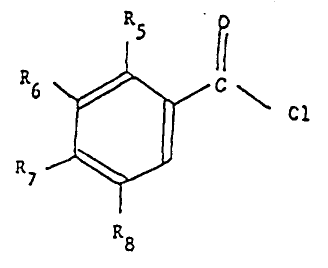 with an allyl p-hydroxybenzoate of formula: 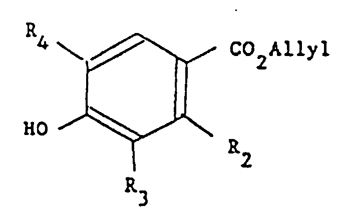 in which:
R₂ to R₈ have the same meanings as those given in Claim 1, in that the allyl ester obtained is converted to the corresponding acid in the presence, as catalyst, of a transition metal complex and in that, if necessary, the corresponding acid chloride is formed and subsequently converted either to an ester by reaction with an alcohol (R₉OH) or to an amide by reaction with an amine

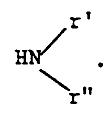

17. Pharmaceutical composition, characterized in that it contains, in an appropriate vehicle for enteral, parenteral, topical or ocular administration, at least one compound of formula (I) according to any one of Claims 1 to 14.

18. Composition according to Claim 17, characterized in that it contains from 0.0001 to approximately 5% by weight of a compound of formula (I).

19. Use of a compound according to any one of Claims 1 to 14 for the preparation of a pharmaceutical composition intended for the treatment of dermatological, rheumatic, respiratory and ophthalmological complaints.

20. Cosmetic composition for body and hair hygiene, characterized in that it contains, in an appropriate cosmetic vehicle, at least one compound of formula (I) according to any one of Claims 1 to 14.

21. Cosmetic composition according to Claim 20, characterized in that it contains the compound of formula (I) at a concentration of between 0.0001 and 0.1% and preferably between 0.001 and 0.01% by weight.

## Patentansprüche

1. Diaromatische Ester, dadurch gekennzeichnet, daß sie unter den folgenden Verbindungen ausgewählt sind:
4-[3-(1-Adamantyl)-4-methoxybenzoyloxy]-3-fluorbenzoesäure,
4-[3-(1-Adamantyl)-4-methoxybenzoyloxy]-3-methoxybenzoesäure,
4-[3-(1-Adamantyl)-4-methoxybenzoyloxy]-isophthalsäure,
4-[3-(1-Adamantyl)-4-acetoxybenzoyloxy]-benzoesäure,
4-[3-(1-Adamantyl)-4-(carboxymethylenoxy)-benzoyloxy]benzoesäure,
4-[3-(1-Adamantyl)-4-(2,3-dihydroxypropyloxy)-benzoyloxy]-benzoesäure,
4-[3-(1-Adamantyl)-4-(methoxycarbonylmethyloxy)-benzoyloxy]-benzoesäure,
sowie die der folgenden allgemeinen Formel entsprechenden Verbindungen: 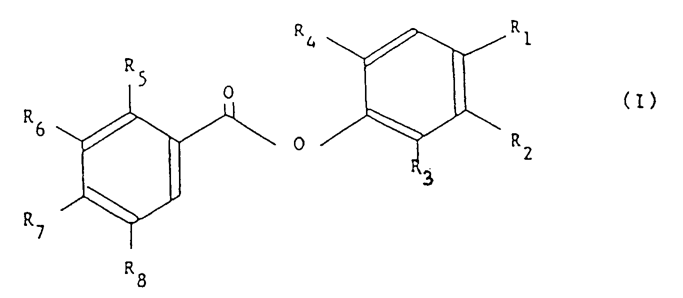 worin:
R₁ die OH-Gruppe, die Gruppen -CH₃, -CH₂OH, -CH(OH)CH₃, -COOR₉, 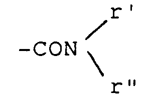 oder SO₂R₁₀ bedeutet,
R₉ ein Wasserstoffatom, einen Alkylrest mit 1 bis 6 Kohlenstoffatomen oder einen Mono- oder Polyhydroxyalkylrest darstellt,
wobei R₁₀ die Hydroxylgruppe, einen Alkylrest mit 1 bis 6 Kohlenstoffatomen oder den Rest 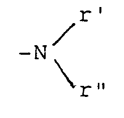 bedeutet,
r' und r'' ein Wasserstoffatom, einen Alkylrest mit 1 bis 6 Kohlenstoffatomen, einen Aryl-, Aralkyl-, Mono- oder Polyhydroxyalkylrest darstellen, oder r' und r'' zusammengenommen einen Heterocyclus bilden,
R₂ ein Wasserstoffatom, einen Alkylrest mit 1 bis 6 Kohlenstoffatomen, den Rest OR₉, ein Fluoratom oder den Rest -CF₃ darstellt,
R₃, R₄ und R₅ ein Wasserstoffatom, ein Fluoratom, die OH-Gruppe, die Reste -CH₃, -OCH₃, -CF₃, -COOH oder -CH₂OH bedeuten,
R₆ und R₈ ein Wasserstoffatom, einen α-substituierten Alkylrest mit 3 bis 15 Kohlenstoffatomen, einen α,α'-disubstituierten Alkylrest mit 4 bis 12 Kohlenstoffatomen, einen Cycloalkylrest mit 3 bis 12 Kohlenstoffatomen, einen mono- oder polycyclischen Cycloalkylrest mit 5 bis 12 Kohlenstoffatomen, dessen Bindungskohlenstoff dreifach substituiert ist, oder den Rest -SR₁₁, -SO₂R₁₁ oder -SOR₁₁ darstellen,
R₁₁ einen Alkylrest mit 1 bis 6 Kohlenstoffatomen oder einen Cycloalkylrest bedeutet, und zwar mit der Maßgabe, daß R₆ und R₈ nicht gleichzeitig ein Wasserstoffatom darstellen,
R₇ ein Wasserstoffatom, einen Alkylrest mit 1 bis 6 Kohlenstoffatomen, einen Alkenylrest, einen Alkenyloxyrest, den Rest -OR₁₂, SR₁₃, SOR₁₄ oder SO₂R₁₄ bedeuten,
R₁₂ ein Wasserstoffatom, einen Alkylrest mit 1 bis 6 Kohlenstoffatomen, einen Alkenylrest, einen Mono- oder Polyhydroxyalkylrest oder das Radikal -(CH₂)ₙ-COR₁₅ bedeutet,
n = 0, 1 oder 2 bedeutet und R₁₅ ein Wasserstoffatom, die OH-Gruppe, einen Alkylrest mit 1 bis 6 Kohlenstoffatomen oder einen Alkoxyrest mit 1 bis 6 Kohlenstoffatomen darstellt,
R₁₃ ein Wasserstoffatom, einen Alkylrest mit 1 bis 6 Kohlenstoffatomen oder einen Aralkylrest bedeutet,
R₁₄ die OH-Gruppe, einen Alkylrest mit 1 bis 6 Kohlenstoffatomen oder einen Aralkylrest mit der Maßgabe bedeutet, daß dann, wenn R₁ -CH₂OH, -CH(OH)CH₃, -COOR₉ oder 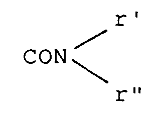 sowie R₂ ein Wasserstoffatom bedeuten,
(i) entweder R₃ und R₄ von einem Wasserstoffatom oder einer -CH₃-Gruppe verschieden sind,
(ii) oder R₇ von dem Rest OR₁₂ verschieden ist und R₆ oder R₈ einen Cycloalkylrest mit mehr als 7 Kohlenstoffatome bedeuten,
(iii) oder R₇ den Rest OR₁₂ darstellt, jedoch R₆ oder R₈ von einem Wasserstoffatom verschieden sind,
(iv) oder R₇ den Rest OR₁₂ bedeutet, jedoch R₅ von einem Wasserstoffatom verschieden ist, mit Ausnahme von Verbindungen gemäß Formel (I), worin R₁ die Methylgruppe bedeutet, R₂ und R₅ ein Wasserstoffatom bedeuten, R₃ und R₄ die Methylgruppe bedeuten, R₆ und R₈ die sekundäre Butylgruppe und R₇ die OH-Gruppe bedeuten.

2. Verbindungen gemäß Anspruch 1, dadurch gekennzeichnet, daß sie in Form von Alkali- oder Erdalkalisalzen oder auch in Form von Zink- oder organischen Aminsalzen vorliegen.

3. Verbindungen gemäß Anspruch 1, dadurch gekennzeichnet, daß der niedere Alkylrest mit 1 bis 6 Kohlenstoffatomen aus der Gruppe ausgewählt ist, welche durch die Methyl-, Ethyl-, Isopropyl-, Butyl- und t-Butylgruppe gebildet ist.

4. Verbindungen gemäß Anspruch 1, dadurch gekennzeichnet, daß der α-substituierte Alkylrest aus der Gruppe ausgewählt ist, welche aus der Isopropyl-, 1-Methylpropyl-, 1-Ethylpropyl-, 1-Methylhexyl-, 1-Methyldecyl- sowie 1-Ethyldodecylgruppe besteht.

5. Verbindungen gemäß Anspruch 1, dadurch gekennzeichnet, daß der α,α'-disubstituierte Alkylrest aus der Gruppe ausgewählt ist, welche aus der t-Butyl-, 1,1-Dimethylpropyl-, 1-Methyl-1-ethylpropyl-, 1-Methyl-1-ethylhexyl- sowie 1,1-Dimethyldecylgruppe besteht.

6. Verbindungen gemäß Anspruch 1, dadurch gekennzeichnet, daß der Monohydroxyalkylrest eine 2-Hydroxyethyl-, 2-Hydroxypropyl- oder 3-Hydroxypropylgruppe darstellt.

7. Verbindungen gemäß Anspruch 1, dadurch gekennzeichnet, daß der Polyhydroxyalkylrest zwischen 3 und 6 Kohlenstoffatome sowie zwischen 2 und 5 Hydroxylgruppen enthält und aus der Gruppe ausgewählt ist, die aus der 2,3-Dihydroxypropyl-, 2,3,4-Trihydroxybutyl-, 2,3,4,5-Tetrahydroxypentyl- sowie der Pentaerythritgruppe ausgewählt ist.

8. Verbindungen gemäß Anspruch 1, dadurch gekennzeichnet, daß der Alkenylrest mit 2 bis 6 Kohlenstoffatomen die Vinyl-, Allyl- sowie 2-Butenylgruppe bedeutet.

9. Verbindungen gemäß Anspruch 1, dadurch gekennzeichnet, daß der Arylrest eine gegebenenfalls durch ein Wasserstoffatom, eine Hydroxylgruppe oder eine Nitrofunktion substituierte Phenylgruppe bedeutet.

10. Verbindungen gemäß Anspruch 1, dadurch gekennzeichnet, daß der Aralkylrest eine Benzylgruppe darstellt oder eine durch mindestens ein Halogenatom, eine Hydroxylgruppe oder eine Nitrofunktion substituierte Phenylethylgruppe darstellt.

11. Verbindungen gemäß Anspruch 1, dadurch gekennzeichnet, daß der mono- oder polycyclische Cycloalkylrest mit 5 bis 12 Kohlenstoffatomen, dessen Bindungskohlenstoff dreifach substituiert ist, die 1-Methylcyclohexyl- oder 1-Adamantylgruppe bedeutet.

12. Verbindungen gemäß Anspruch 1, dadurch gekennzeichnet, daß die Reste r' und r'' zusammengenommen einen Heterocyclus bilden, welcher aus der Gruppe ausgewählt ist, die aus einer Piperidin-, Morpholin-, Pyrrolidin- oder Piperazingruppe besteht, welche jeweils gegebenenfalls in der 4-Stellung durch einen C₁-C₆-Alkylrest oder einen Mono- oder Polyhydroxyalkylrest substituiert sind.

13. Verbindungen gemäß einem der vorstehenden Ansprüche, dadurch gekennzeichnet, daß sie aus der Gruppe ausgewählt sind, welche aus den folgenden Verbindungen besteht:
4-[3-(1-Adamantyl)-4-methoxybenzoyloxy]-2-fluorbenzoesäure,
4-[3-(1-Adamantyl)-4-methoxybenzoyloxy]-2-methylbenzoesäure,
4-[3-(1-Adamantyl)-4-methoxybenzoyloxy]-2-hydroxybenzoesäure,
4-[3-(1-Adamantyl)-4-methoxybenzoyloxy]-2-methoxybenzoesäure,
4-[3-(1-Adamantyl)-4-methoxybenzoyloxy]-2-trifluormethylbenzoesäure,
Methyl-4-[3-(1-adamantyl)-4-methoxybenzoyloxy]-2-hydroxybenzoat,
4-[5-(1-Adamantyl)-2-fluor-4-methoxybenzoyloxy]-benzoesäure,
4-[5-(1-Adamantyl)-2,4-dimethoxybenzoyloxy]-benzoesäure,
4-[3-(1-Adamantyl)-benzoyloxy]-benzoesäure,
4-[3,5-Di-t-butyl-4-hydroxybenzoyloxy]-benzoesäure,
4-[3-(1-Adamantyl)-4-vinylbenzoyloxy]-benzoesäure,
4-[3-(1-Adamantyl)-4-ethylbenzoyloxy]-benzoesäure,
4-[3-(1-Adamantyl)-4-butylbenzoyloxy]-benzoesäure,
4-[3-(1-Adamantyl)-4-allyloxybenzoyloxy]-benzoesäure,
4-[3-(1-Adamantyl)-4-methoxybenzoyloxy]-benzolsulfonsäureamid,
4-Hydroxyphenyl-3-(1-adamantyl)-4-methoxybenzoat,
Phenyl-3-(1-adamantyl)-4-methoxybenzoat,
4-Methylphenyl-3-(1-adamantyl)-4-methoxybenzoat,
4-[3-(1-Adamantyl)-4-methoxybenzoyloxy]-3-hydroxymethylbenzoesäure,
4-[3-(1-Adamantyl)-4-methylthiobenzoyloxy]-benzoesäure,
4-[3-(1-Adamantyl)-4-methylsulfonbenzoyloxy]-benzoesäure.

14. Verbindungen gemäß einem der vorstehenden Ansprüche, dadurch gekennzeichnet, daß sie vorzugsweise der folgenden allgemeinen Formel entsprechen: 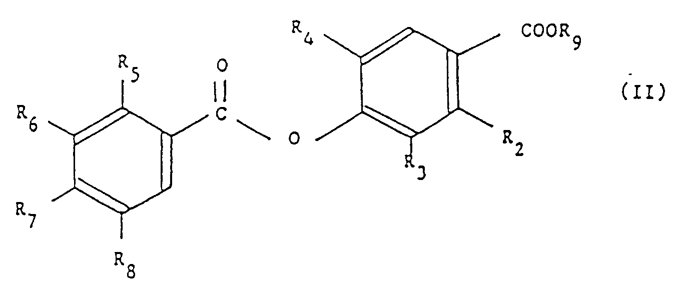 worin:
(i) entweder R₃ bis R₉ der Definition gemäß Anspruch 1 entsprechen und R₂ einen Alkylrest mit 1 bis 6 Kohlenstoffatomen, den Rest OR₉, ein Fluoratom oder den Rest -CF₃ bedeutet,
(ii) oder R₂ bis R₄ sowie R₆ bis R₉ der Definition gemäß Anspruch 1 entsprechen und R₅ ein Fluoratom darstellt.

15. Verfahren zur Herstellung von Verbindungen der Formel (I) gemäß einem der Ansprüche 1 bis 14, worin R₁ = H oder -CH₃ bedeutet, dadurch gekennzeichnet, daß man in einem organischen Lösungsmittel in Anwesenheit eines tertiären Amins oder eines Alkalihydrids eine aktivierte Form einer substituierten Benzoesäure, insbesondere eines Säurechlorids der Formel: 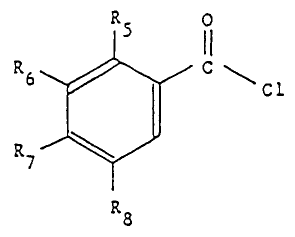 mit einem p-Hydroxybenzolderivat der Formel: 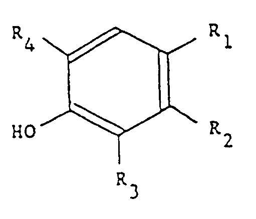 reagieren läßt, worin:
R₁ ein Wasserstoffatom oder die -CH₃-Gruppe bedeutet, und
R₂ bis R₈ dieselben Bedeutungen aufweisen, wie sie in Anspruch 1 definiert sind, wobei die Reaktion bei Raumtemperatur und unter Rühren durchgeführt wird.

16. Verfahren zur Herstellung der Verbindung der Formel (I) gemäß einem der Ansprüche 1 bis 14, worin
R₁ = -COOR₉ oder 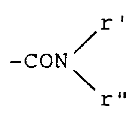 bedeutet, dadurch gekennzeichnet, daß man in Anwesenheit eines tertiären Amins ein Säurechlorid der Formel: 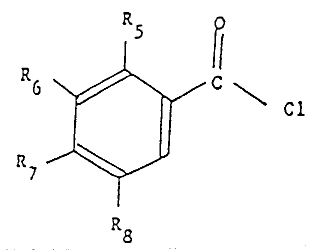 mit einer Allyl-o-hydroxybenzoatverbindung der Formel: 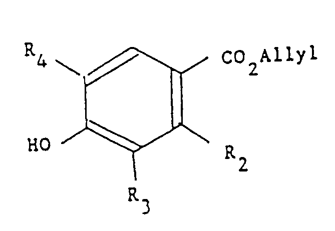 zur Reaktion bringt, worin:
R₂ bis R₈ dieselben Bedeutungen aufweisen, wie sie in Anspruch 1 definiert sind, und daß man den erhaltenen Allylester in Anwesenheit eines Übergangsmetallkomplexes als Katalysator in die entsprechende Säure überführt, und daß man die Reaktion erforderlichenfalls bis zur Bildung des entsprechenden Säurechlorids und der nachfolgenden Umwandlung des Chlorids in den Ester durch die Reaktion eines Alkohols (R₉OH) oder in das Amid
durch Reaktion eines Amins 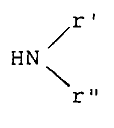 fortführt.

17. Arzneimittel, dadurch gekennzeichnet, daß es in einem geeigneten Träger zur enteralen, parenteralen, topischen oder ophthalmologischen Verabreichung mindestens eine Verbindung der Formel (I) gemäß einem der Ansprüche 1 bis 14 enthält.

18. Arzneimittel gemäß Anspruch 17, dadurch gekennzeichnet, daß es zwischen 0,0001 und etwa 5 Gew.-% einer Verbindung gemäß Formel (I) enthält.

19. Verwendung einer Verbindung gemäß einem der Ansprüche 1 bis 14 zur Herstellung eines Arzneimittels, welches zur Behandlung dermatologischer oder rheumatischer Affektionen bzw. zur Behandlung von Störungen des Atemtrakts oder der Augen bestimmt ist.

20. Kosmetische Zubereitung für die Körper- oder Haarhygiene, dadurch gekennzeichnet, daß sie in einem geeigneten kosmetischen Träger mindestens eine Verbindung der Formel (I) gemäß einem der Ansprüche 1 bis 14 enthält.

21. Kosmetische Zubereitung gemäß Anspruch 20, dadurch gekennzeichnet, daß sie die Verbindung der Formel (I) in einer Konzentration zwischen 0,0001 und 0,1 Gew.-%, vorzugsweise in einer Konzentration zwischen 0,001 und 0,01 Gew.-%, enthält.
